# EUROPEAN PATENT SPECIFICATION

(11) **EP 4 103 178 B1**
(45) Date of publication and mention of the grant of the patent: **24.12.2025**
(21) Application number: 21753606.9
(22) Date of filing: 10.02.2021
(51) Int. Cl.: A61K 31/445, A61P 9/10, A61P 13/12, A61P 3/00

(54) **METHODS OF TREATING FABRY DISEASE**
VERFAHREN ZUM BEHANDELN DER FABRY-KRANKHEIT
MÉTHODES DE TRAITEMENT DE LA MALADIE DE FABRY

(30) Priority: 10.02.2020 US 202062972392 P
(43) Date of publication of application: 21.12.2022
(73) Proprietor: Amicus Therapeutics, Inc., Philadelphia, PA 19104 (US)
(72) Inventor: SKUBAN, Nina, Philadelphia, Pennsylvania 19104 (US)
(74) Representative: Hoffmann Eitle
(86) International application number: PCT/US2021/017446
(87) International publication number: WO 2021/163183

(56) References cited:
- US-A1- 2016 324 839
- US-B1- 6 274 597
- ANONYMOUS: "EUROPEAN MEDICINES AGENCY (EMA) Galafold", 9 June 2016 (2016-06-09), Internet, pages 1 - 117, XP093061474, Retrieved from the Internet <URL:https://www.ema.europa.eu/en/medicines/human/EPAR/galafold> [retrieved on 20230706]
- ANONYMOUS: "Clinical Review Report - Migalastat (Galafold)", NCBI, 1 February 2018 (2018-02-01), pages 1 - 98, XP093122971, Retrieved from the Internet <URL:https://www.ncbi.nlm.nih.gov/books/NBK533664/> [retrieved on 20240123]
- LOURENCO CHARLES ET AL: "Abstracts presented at the 13th International Congress of Inborn Errors of Metabolism - ICIEM 2017", vol. 5, 1 January 2017 (2017-01-01), XP093123210, ISSN: 2326-4098, Retrieved from the Internet <URL:http://journals.sagepub.com/doi/full-xml/10.1177/2326409817722292> DOI: 10.1177/2326409817722292
- AMICUS THERAPEUTICS: "Safety, Pharmacokinetics, Pharmacodynamics, and Efficacy of Migalastat in Pediatric Subjects (Aged 12 to <18 Years) (v10)", CLINICALTRIALS.GOV ARCHIVE, 11 December 2019 (2019-12-11), XP055849379, Retrieved from the Internet <URL:https://clinicaltrials.gov/ct2/history/NCT03500094?V_10=View#StudyPageTop> [retrieved on 20211008]
- SCHIFFMANN RAPHAEL, BICHET DANIEL G., JOVANOVIC ANA, HUGHES DERRALYNN A., GIUGLIANI ROBERTO, FELDT-RASMUSSEN ULLA, SHANKAR SUMA P.: "Migalastat improves diarrhea in patients with Fabry disease: clinical-biomarker correlations from the phase 3 FACETS trial", ORPHANET JOURNAL OF RARE DISEASES, vol. 13, no. 1, 68, 27 April 2018 (2018-04-27), XP055849383, DOI: 10.1186/s13023-018-0813-7

## Description

### TECHNICAL FIELD

Principles and embodiments of the present invention relate to migalastat or salt thereof for use in a method of reducing the risk of composite clinical outcomes (CCO) in a female patient having Fabry disease as defined in the appended claims.

### CROSS-REFERENCE TO SEQUENCE LISTING

The Sequence Listing identified as "AT20-01_Sequence_Listing.txt" (21,991 bytes), created February 10, 2021, is hereby incorporated by reference.

### BACKGROUND

Fabry disease is a progressive, X-linked inborn error of glycosphingolipid metabolism caused by a deficiency in the lysosomal enzyme α-galactosidase A (α-Gal A) as a result of mutations in the α-Gal A gene (GLA). Despite being an X-linked disorder, females can express varying degrees of clinical manifestations. Fabry is a rare disease with incidence estimated between 1 in 40,000 males to 1 in 117,000 in the general population. Moreover, there are variants of later onset phenotype of Fabry disease that can be under-diagnosed, as they do not present with classical signs and symptoms. This, and newborn screening for Fabry disease, suggests that the actual incidence of Fabry disease can be higher than currently estimated.

Untreated, life expectancy in Fabry patients is reduced and death usually occurs in the fourth or fifth decade because of vascular disease affecting the kidneys, heart and/or central nervous system. The enzyme deficiency leads to intracellular accumulation of the substrate, globotriaosylceramide (GL-3) in the vascular endothelium and visceral tissues throughout the body. Gradual deterioration of renal function and the development of azotemia, due to glycosphingolipid deposition, usually occur in the third to fifth decades of life, but can occur as early as in the second decade. Renal lesions are found in both hemizygous (male) and heterozygous (female) patients.

Cardiac disease as a result of Fabry disease occurs in most males and many females. Early cardiac findings include left ventricular enlargement, valvular involvement and conduction abnormalities. Mitral insufficiency is the most frequent valvular lesion typically present in childhood or adolescence. Cerebrovascular manifestations result primarily from multifocal small-vessel involvement and can include thromboses, transient ischemic attacks, basilar artery ischemia and aneurysm, seizures, hemiplegia, hemianesthesia, aphasia, labyrinthine disorders, or cerebral hemorrhages. Average age of onset of cerebrovascular manifestations is 33.8 years. Personality change and psychotic behavior can manifest with increasing age.

One approved therapy for treating Fabry disease is enzyme replacement therapy (ERT), which typically involves intravenous, infusion of a purified form of the corresponding wild-type protein. Two α-Gal A products are currently available for the treatment of Fabry disease: agalsidase alfa (Replagal^{®}, Shire Human Genetic Therapies) and agalsidase beta (Fabrazyme^{®}; Sanofi Genzyme Corporation). While ERT is effective in many settings, the treatment also has limitations. ERT has not been demonstrated to decrease the risk of stroke, cardiac muscle responds slowly, and GL-3 elimination from some of the cell types of the kidneys is limited. Some patients also develop immune reactions to ERT.

Accordingly, there remains a need for therapies for the treatment of Fabry disease. - The Public European Assessment Report of Galafold (migalastat) can be accessed at https://www.ema.europa.eu/en/medicines/human/EPAR/galafold, and a Clinical Review Report at https://www.ncbi.nlm.nih.gov/books/NBK533664/.

### SUMMARY

The present invention pertains to migalastat or salt thereof for use in a method of reducing the risk of composite clinical outcomes (CCO) in a female patient having Fabry disease, the method comprising administering to the patient a formulation comprising an effective amount of migalastat or salt thereof every other day for at least 18 months, wherein the effective amount is about 100 mg to about 150 mg free base equivalent (FBE).

The CCO comprises renal events, cardiac events, cerebrovascular events and death. In one or more embodiments, the renal events comprise one or more of: a decrease in eGFR_{CKD-EPI} ≥15 mL/min/1.73 m², with the decreased eGFR <90 mL/min/1.73 m² relative to baseline; or an increase in 24-hour urine protein ≥33%, with elevated protein ≥300 mg relative to baseline. In one or more embodiments, the cardiac events comprise one or more of: myocardial infarction; unstable cardiac angina; new symptomatic arrhythmia requiring antiarrhythmic medication, direct current cardioversion, pacemaker, or defibrillator implantation; or congestive heart failure [New York Association Class III or IV]. In one or more embodiments, the cerebrovascular events comprise one or more of stroke or transient ischemic attack.

In one or more embodiments, the migalastat or salt thereof enhances α-Gal A activity.

In one or more embodiments, the patient is administered about 123 mg FBE of the migalastat or salt thereof every other day.

In one or more embodiments, the patient is administered about 123 mg of migalastat free base every other day.

In one or more embodiments, the patient is administered about 150 mg of migalastat hydrochloride every other day.

In one or more embodiments, the formulation comprises an oral dosage form. In one or more embodiments, the oral dosage form comprises a tablet, a capsule or a solution.

In one or more embodiments, the migalastat or salt thereof is administered for at least 2 years.

In one or more embodiments, the migalastat or salt thereof is administered for at least 3 years.

In one or more embodiments, the migalastat or salt thereof is administered for at least 4 years.

In one or more embodiments, the CCO incidence rate for a group of patients on migalastat therapy for 18 months is less than 1.0 per patient•year. In one or more embodiments, the CCO incidence rate for a group of patients on migalastat therapy for 18 months is less than 0.5 per patient•year.

In one or more embodiments, the patient is an ERT-naïve patient.

In one or more embodiments, the patient is an ERT-experienced patient.

In one or more embodiments, the patient has a HEK assay amenable mutation in α-galactosidase A. In one or more embodiments, the mutation is disclosed in a pharmacological reference table. In one or more embodiments, the pharmacological reference table is provided in a product label for a migalastat product approved for the treatment of Fabry disease. In one or more embodiments, the pharmacological reference table is provided in a product label for GALAFOLD^{®}. In one or more embodiments, the pharmacological reference table is provided at a website. In one or more embodiments, the website is one or more of www.galafoldamenabilitytable.com or www.fabrygenevariantsearch.com.

Also disclosed herein is a method of assessing gastrointestinal (GI) outcomes in a patient having Fabry disease, the method comprising: assessing the patient's disease-related GI symptom severity; assessing the patient's frequency of bowel movements; assessing the patient's frequency of diarrhea; and assessing the consistency of the patient's diarrhea.

In one or more aspects, assessing the patient's disease-related GI symptom severity comprises assessing the severity of one or more of: bloating, stomach pain, cramping, nausea, acid reflux, heartburn, constipation, or diarrhea.

In one or more aspects, assessing the patient's disease-related GI symptom severity comprises assessing the severity of one or more of: the worst bloating over a time period; the worst stomach pain over at time period; the worst cramping over a time period; the worst nausea over a time period; the worst nausea over a time period; the worst nausea over a time period; the worst nausea over a time period; or the worst nausea over a time period.

In one or more aspects, the GI outcomes are assessed based on a 24-hour time period. In other aspects, the assessments are based on a 6-hour, 8-hour, 12-hour, 36-hour, 48-hour, 3-day, 4-day, 5-day, 6-day or 7-day time period.

In one or more aspects, the GI outcomes are assessed based on patient-reported symptoms.

In one or more aspects, each item is assessed using a score on a 0-10 scale.

Also disclosed herein is a method of evaluating a treatment therapy for Fabry disease, the method comprising assessing the GI at baseline, assessing the GI outcome after a period of the treatment therapy, and comparing the GI outcome at baseline to the GI outcome after the period of the treatment therapy.

Also disclosed herein is a method of treating Fabry disease, the method comprising assessing the GI to obtain a first GI score, initiating or continuing a treatment therapy for Fabry disease for a time period, assessing the GI outcome after a period of the treatment therapy after the time period of treatment therapy to obtain a second GI outcome score, and comparing the first GI outcome score and the second GI outcome score.

In one or more aspects, the treatment therapy comprises ERT.

In one or more aspects, the treatment therapy comprises substrate reduction therapy.

In one or more aspects, the treatment therapy comprises gene therapy.

In one or more aspects, the treatment therapy comprises pharmacological chaperone therapy. In one or more aspects, the pharmacological chaperone therapy comprises administration of an effective amount of migalastat or a salt thereof. In one or more aspects, the migalastat or salt thereof is administered every other day. In one or more aspects embodiments, the effective amount is about 100 mg to about 150 mg FBE.

In one or more aspects, the patient is administered about 123 mg FBE of the migalastat or salt thereof every other day.

In one or more aspects, the patient is administered about 123 mg of migalastat free base every other day.

In one or more aspects, the patient is administered about 150 mg of migalastat hydrochloride every other day.

In one or more aspects, the formulation comprises an oral dosage form. In one or more aspects, the oral dosage form comprises a tablet, a capsule or a solution.

In one or more aspects, the migalastat or salt thereof is administered for at least 2 years.

In one or more aspects, the migalastat or salt thereof is administered for at least 3 years.

In one or more aspects, the migalastat or salt thereof is administered for at least 4 years.

In one or more aspects, the patient is male.

In one or more aspects, the patient is female.

In one or more aspects, the patient is an ERT-naïve patient.

In one or more aspects, the patient is an ERT-experienced patient.

In one or more aspects, the patient has a HEK assay amenable mutation in α-galactosidase A. In one or more aspects, the mutation is disclosed in a pharmacological reference table. In one or more aspects, the pharmacological reference table is provided in a product label for a migalastat product approved for the treatment of Fabry disease. In one or more aspects, the pharmacological reference table is provided in a product label for GALAFOLD^{®}. In one or more aspects, the pharmacological reference table is provided at a website. In one or more aspects, the website is one or more of www.galafoldamenabilitytable.com or www.fabrygenevariantsearch.com.

Also disclosed herein is a method of evaluating a treatment therapy for Fabry disease, the method comprising assessing one or more parameters in a patient population treated with the treatment therapy, wherein the one or more parameters comprise one or more of: incidence of Fabry signs and symptoms; renal parameters; and cardiac parameters; and assessing the one or more parameters in an untreated patient population.

In one or more aspects, the Fabry signs and symptoms comprise one or more of: acroparasthesias; GI signs and symptoms; hearing loss; corneal whirling; angiokeratomas; hypohidrosis; pulmonary changes; lymphedema; or brain MRI changes.

In one or more aspects, the renal parameters comprise one or more of eGFR_{CKD-EPI}; creatinine levels; urine protein levels; or incidence of detectable urine protein.

In one or more aspects, the cardiac parameters comprise one or more of left ventricular mass index (LVMi) or incidence of left ventricular hypertrophy.

In one or more aspects, the method further comprises assessing the one or more parameters in a patient population treated with a different treatment therapy for Fabry disease.

In one or more aspects, the method further comprises assessing the patient ages in each of the patient populations.

In one or more aspects, the method further comprises assessing the patient genotypes in each of the patient populations.

In one or more aspects, the method further comprises assessing the patient genders in each of the patient populations.

In one or more aspects, the treatment therapy comprises ERT.

In one or more aspects, the treatment therapy comprises substrate reduction therapy.

In one or more aspects, the treatment therapy comprises gene therapy.

In one or more aspects, the treatment therapy comprises pharmacological chaperone therapy. In one or more aspects, the pharmacological chaperone therapy comprises administration of an effective amount of migalastat or a salt thereof. In one or more aspects, the migalastat or salt thereof is administered every other day. In one or more aspects, the effective amount is about 100 mg to about 150 mg FBE.

In one or more aspects, the patient is administered about 123 mg FBE of the migalastat or salt thereof every other day.

In one or more aspects, the patient is administered about 123 mg of migalastat free base every other day.

In one or more aspects, the patient is administered about 150 mg of migalastat hydrochloride every other day.

In one or more aspects, the formulation comprises an oral dosage form. In one or more aspects, the oral dosage form comprises a tablet, a capsule or a solution.

In one or more aspects, the migalastat or salt thereof is administered for at least 2 years.

In one or more aspects, the migalastat or salt thereof is administered for at least 3 years.

In one or more aspects, the migalastat or salt thereof is administered for at least 4 years.

In one or more aspects, the patient is male.

In one or more aspects, the patient is female.

In one or more aspects, the patient is an ERT-naïve patient.

In one or more aspects, the patient is an ERT-experienced patient.

In one or more aspects, the patient has a HEK assay amenable mutation in α-galactosidase A. In one or more aspects, the mutation is disclosed in a pharmacological reference table. In one or more embodiments, the pharmacological reference table is provided in a product label for a migalastat product approved for the treatment of Fabry disease. In one or more aspects, the pharmacological reference table is provided in a product label for GALAFOLD^{®}. In one or more aspects, the pharmacological reference table is provided at a website. In one or more aspects, the website is one or more of www.galafoldamenabilitytable.com or www.fabrygenevariantsearch.com.

In one or more aspects, the method further comprises assessing the one or more parameters in a patient population treated with ERT.

In one or more aspects, the one or more parameters are assessed for a time period of at least one year.

### BRIEF DESCRIPTION OF THE DRAWINGS

Further features of the present invention will become apparent from the following written description and the accompanying figures, in which:
FIGS. 1A-E show the full DNA sequence of the human wild-type GLA gene (SEQ ID NO: 1);
FIG. 2 shows the wild-type α-Gal A protein (SEQ ID NO: 2);
FIG. 3 shows the nucleic acid sequence encoding the wild-type α-Gal A protein (SEQ ID NO: 3);
FIGS. 4A and 4B show the Fabry disease history of patients currently enrolled in the followME registry (Safety Population); and
FIG. 5A shows Fabry disease signs and symptoms in patients currently enrolled in the followME registry and FIG. 5B shows the median age at first occurrence of Fabry signs and symptoms (Safety Population) by gender.

### DETAILED DESCRIPTION

Before describing several exemplary embodiments of the invention, it is to be understood that the invention is not limited to the details of construction or process steps set forth in the following description. The invention is capable of other embodiments and of being practiced or being carried out in various ways.

The present invention pertains to the administration of migalastat for the treatment of Fabry disease as defined in the appended claims.

### Definitions

The terms used in this specification generally have their ordinary meanings in the art, within the context of this invention and in the specific context where each term is used. Certain terms are discussed below, or elsewhere in the specification, to provide additional guidance to the practitioner in describing the compositions and methods of the invention and how to make and use them.

The term "Fabry disease" refers to an X-linked inborn error of glycosphingolipid catabolism due to deficient lysosomal α-Gal A activity. This defect causes accumulation of the substrate globotriaosylceramide ("GL-3", also known as Gb3 or ceramide trihexoside) and related glycosphingolipids in vascular endothelial lysosomes of the heart, kidneys, skin, and other tissues. Another substrate of the enzyme is plasma globotriaosylsphingosine ("plasma lyso-Gb₃").

The term "atypical Fabry disease" refers to patients with primarily cardiac manifestations of the α-Gal A deficiency, namely progressive GL-3 accumulation in myocardial cells that leads to significant enlargement of the heart, particularly the left ventricle.

A "carrier" is a female who has one X chromosome with a defective α-Gal A gene and one X chromosome with the normal gene and in whom X chromosome inactivation of the normal allele is present in one or more cell types. A carrier is often diagnosed with Fabry disease.

A "patient" refers to a subject who has been diagnosed with or is suspected of having a particular disease. The patient may be human or animal.

A "Fabry patient" refers to an individual who has been diagnosed with or suspected of having Fabry disease and has a mutated α-Gal A as defined further below. Characteristic markers of Fabry disease can occur in male hemizygotes and female carriers with the same prevalence, although females typically are less severely affected.

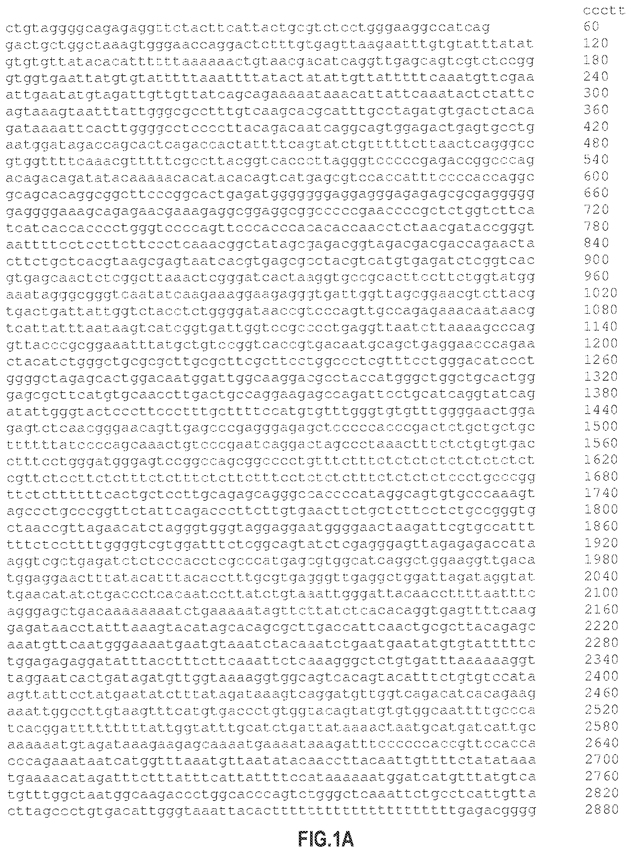

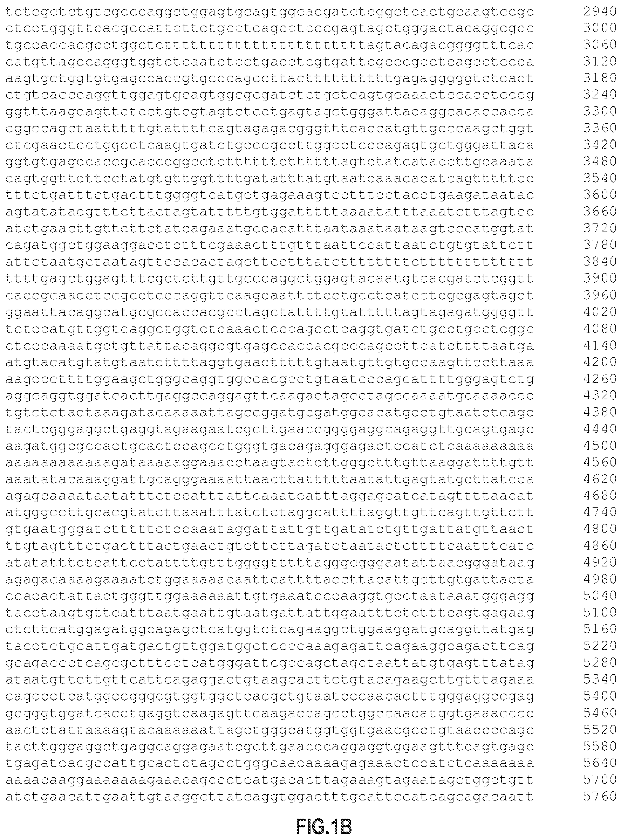

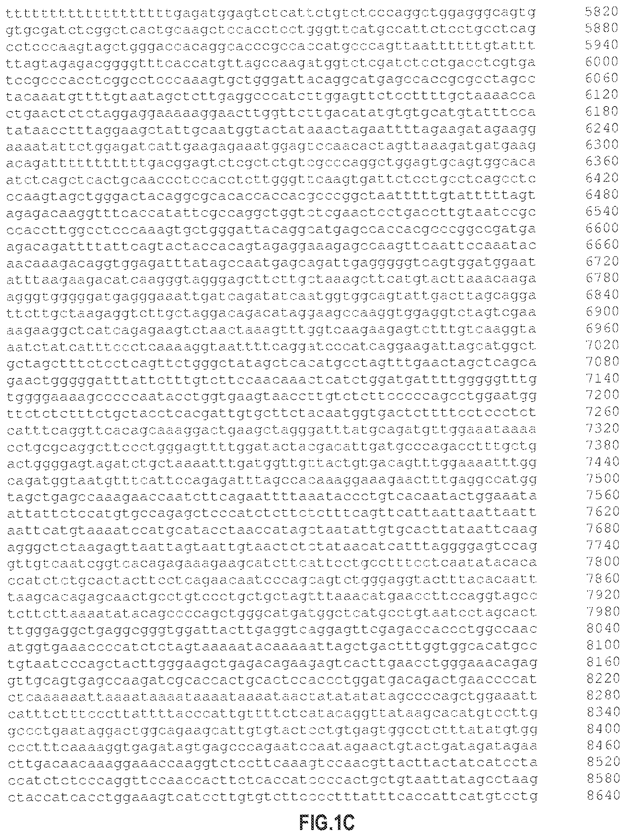

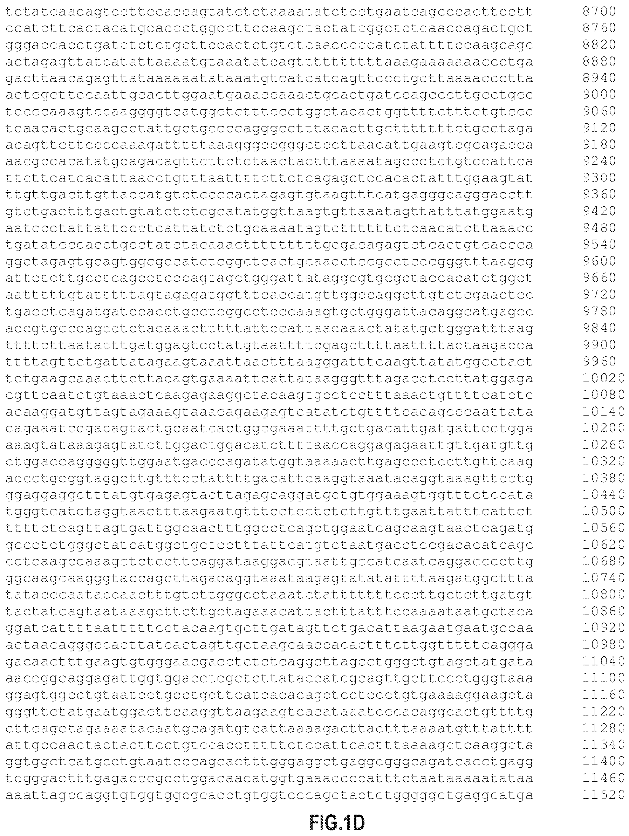

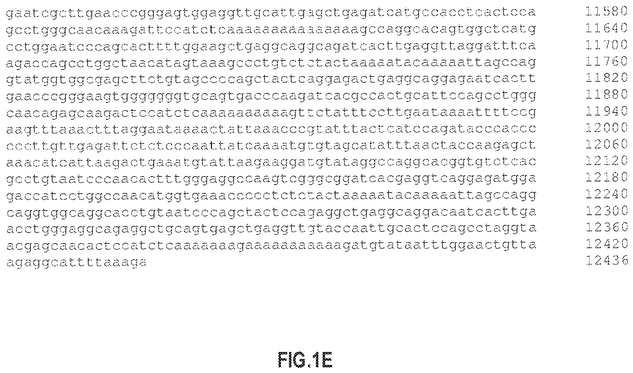

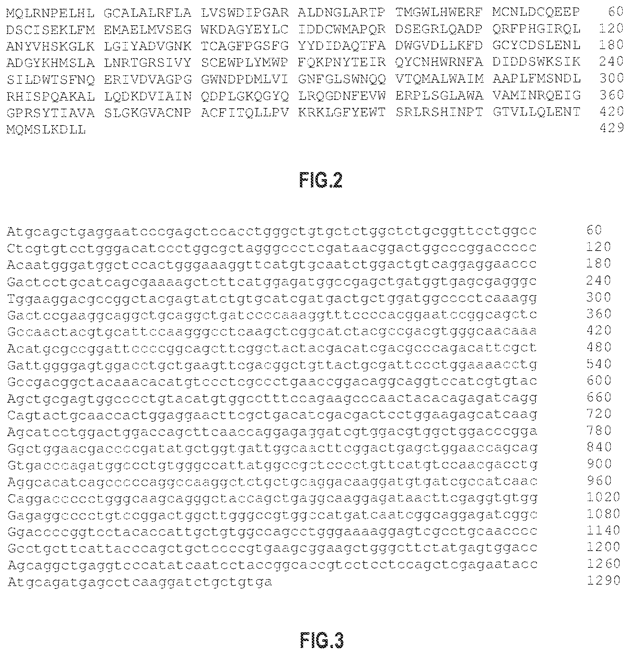

Human α-galactosidase A (α-Gal A) refers to an enzyme encoded by the human GLA gene. The full DNA sequence of α-Gal A, including introns and exons, is available in GenBank Accession No. X14448.1 and shown in FIG. 1A-E (SEQ ID NO: 1). The human α-Gal A enzyme consists of 429 amino acids and is available in GenBank Accession Nos. X14448.1 and U78027.1 and shown in FIG.2 (SEQ ID NO: 2). The nucleic acid sequence that only includes the coding regions (i.e. exons) of SEQ ID NO: 1 is shown in FIG. 3 (SEQ ID NO: 3).

The term "mutant protein" includes a protein which has a mutation in the gene encoding the protein which results in the inability of the protein to achieve a stable conformation under the conditions normally present in the endoplasmic reticulum (ER). The failure to achieve a stable conformation results in a substantial amount of the enzyme being degraded, rather than being transported to the lysosome. Such a mutation is sometimes called a "conformational mutant." Such mutations include, but are not limited to, missense mutations, and in-frame small deletions and insertions.

As used herein in one embodiment, the term "mutant α-Gal A" includes an α-Gal A which has a mutation in the gene encoding α-Gal A which results in the inability of the enzyme to achieve a stable conformation under the conditions normally present in the ER. The failure to achieve a stable conformation results in a substantial amount of the enzyme being degraded, rather than being transported to the lysosome.

As used herein, the term "pharmacological chaperone" ("PC") refers to any molecule including a small molecule, protein, peptide, nucleic acid, carbohydrate, etc. that specifically binds to a protein and has one or more of the following effects: (i) enhances the formation of a stable molecular conformation of the protein; (ii) induces trafficking of the protein from the ER to another cellular location, preferably a native cellular location, *i.e.,* prevents ER-associated degradation of the protein; (iii) prevents aggregation of misfolded proteins; and/or (iv) restores or enhances at least partial wild-type function and/or activity to the protein. A compound that specifically binds to *e.g.,* α-Gal A, means that it binds to and exerts a chaperone effect on the enzyme and not a generic group of related or unrelated enzymes. More specifically, this term does not refer to endogenous chaperones, such as BiP, or to non-specific agents which have demonstrated non-specific chaperone activity against various proteins, such as glycerol, DMSO or deuterated water, *i.e.,* chemical chaperones. In the present invention, the PC is migalastat or a salt thereof. In another embodiment, the PC is migalastat free base (*e.g.,* 123 mg of migalastat free base). In yet another embodiment, the PC is a salt of migalastat (*e.g.,* 150 mg of migalastat HCl).

A "competitive inhibitor" of an enzyme can refer to a compound which structurally resembles the chemical structure and molecular geometry of the enzyme substrate to bind the enzyme in approximately the same location as the substrate. Thus, the inhibitor competes for the same active site as the substrate molecule, thus increasing the Km. Competitive inhibition is usually reversible if sufficient substrate molecules are available to displace the inhibitor, *i.e.,* competitive inhibitors can bind reversibly. Therefore, the amount of enzyme inhibition depends upon the inhibitor concentration, substrate concentration, and the relative affinities of the inhibitor and substrate for the active site.

As used herein, the term "specifically binds" refers to the interaction of a pharmacological chaperone with a protein such as α-Gal A, specifically, an interaction with amino acid residues of the protein that directly participate in contacting the pharmacological chaperone. A pharmacological chaperone specifically binds a target protein, *e.g.,* α-Gal A, to exert a chaperone effect on the protein and not a generic group of related or unrelated proteins. The amino acid residues of a protein that interact with any given pharmacological chaperone may or may not be within the protein's "active site." Specific binding can be evaluated through routine binding assays or through structural studies, *e.g*., co-crystallization, NMR, and the like. The active site for α-Gal A is the substrate binding site.

"Deficient α-Gal A activity" refers to α-Gal A activity in cells from a patient which is below the normal range as compared (using the same methods) to the activity in normal individuals not having or suspected of having Fabry or any other disease (especially a blood disease).

As used herein, the terms "enhance α-Gal A activity" or "increase α-Gal A activity" refer to increasing the amount of α-Gal A that adopts a stable conformation in a cell contacted with a pharmacological chaperone specific for the α-Gal A, relative to the amount in a cell (preferably of the same cell-type or the same cell, *e.g.,* at an earlier time) not contacted with the pharmacological chaperone specific for the α-Gal A. This term also refers to increasing the trafficking of α-Gal A to the lysosome in a cell contacted with a pharmacological chaperone specific for the α-Gal A, relative to the trafficking of α-Gal A not contacted with the pharmacological chaperone specific for the protein. These terms refer to both wild-type and mutant α-Gal A. In one embodiment, the increase in the amount of α-Gal A in the cell is measured by measuring the hydrolysis of an artificial substrate in lysates from cells that have been treated with the PC. An increase in hydrolysis is indicative of increased α-Gal A activity.

The term "α-Gal A activity" refers to the normal physiological function of a wild-type α-Gal A in a cell. For example, α-Gal A activity includes hydrolysis of GL-3.

A "responder" is an individual diagnosed with or suspected of having a lysosomal storage disorder (LSD), such, for example Fabry disease, whose cells exhibit sufficiently increased α-Gal A activity, respectively, and/or amelioration of symptoms or enhancement in surrogate markers, in response to contact with a PC. Non-limiting examples of enhancements in surrogate markers for Fabry are lyso-GB3 and those disclosed in US Patent Application Publication No. U.S. 2010/0113517.

Non-limiting examples of improvements in surrogate markers for Fabry disease disclosed in U.S. 2010/0113517 include increases in α-Gal A levels or activity in cells (*e.g.,* fibroblasts) and tissue; reductions in of GL-3 accumulation; decreased plasma concentrations of homocysteine and vascular cell adhesion molecule-1 (VCAM-1); decreased GL-3 accumulation within myocardial cells and valvular fibrocytes; reduction in plasma lyso-Gb₃; reduction in cardiac hypertrophy (especially of the left ventricle), amelioration of valvular insufficiency, and arrhythmias; amelioration of proteinuria; decreased urinary concentrations of lipids such as CTH, lactosylceramide, ceramide, and increased urinary concentrations of glucosylceramide and sphingomyelin; the absence of laminated inclusion bodies (Zebra bodies) in glomerular epithelial cells; improvements in renal function; mitigation of hypohidrosis; the absence of angiokeratomas; and improvements in hearing abnormalities such as high frequency sensorineural hearing loss progressive hearing loss, sudden deafness, or tinnitus.

The dose that achieves one or more of the aforementioned responses is a "therapeutically effective dose."

The phrase "pharmaceutically acceptable" refers to molecular entities and compositions that are physiologically tolerable and do not typically produce untoward reactions when administered to a human. In some embodiments, as used herein, the term "pharmaceutically acceptable" means approved by a regulatory agency of the Federal or a state government or listed in the U.S. Pharmacopoeia or other generally recognized pharmacopoeia for use in animals, and more particularly in humans. The term "carrier" in reference to a pharmaceutical carrier refers to a diluent, adjuvant, excipient, or vehicle with which the compound is administered. Such pharmaceutical carriers can be sterile liquids, such as water and oils. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E. W. Martin, 18th Edition, or other editions.

As used herein, the term "isolated" means that the referenced material is removed from the environment in which it is normally found. Thus, an isolated biological material can be free of cellular components, *i.e.,* components of the cells in which the material is found or produced. In the case of nucleic acid molecules, an isolated nucleic acid includes a PCR product, an mRNA band on a gel, a cDNA, or a restriction fragment. In another embodiment, an isolated nucleic acid is preferably excised from the chromosome in which it may be found, and more preferably is no longer joined to non-regulatory, non-coding regions, or to other genes, located upstream or downstream of the gene contained by the isolated nucleic acid molecule when found in the chromosome. In yet another embodiment, the isolated nucleic acid lacks one or more introns. Isolated nucleic acids include sequences inserted into plasmids, cosmids, artificial chromosomes, and the like. Thus, in a specific embodiment, a recombinant nucleic acid is an isolated nucleic acid. An isolated protein may be associated with other proteins or nucleic acids, or both, with which it associates in the cell, or with cellular membranes if it is a membrane-associated protein. An isolated organelle, cell, or tissue is removed from the anatomical site in which it is found in an organism. An isolated material may be, but need not be, purified.

The term "enzyme replacement therapy" or "ERT" refers to the introduction of a non-native, purified enzyme into an individual having a deficiency in such enzyme. The administered protein can be obtained from natural sources or by recombinant expression (as described in greater detail below). The term also refers to the introduction of a purified enzyme in an individual otherwise requiring or benefiting from administration of a purified enzyme, *e.g*., suffering from enzyme insufficiency. The introduced enzyme may be a purified, recombinant enzyme produced *in vitro,* or protein purified from isolated tissue or fluid, such as, *e.g.,* placenta or animal milk, or from plants.

The term "ERT-naïve patient" refers to a Fabry patient that has never received ERT or has not received ERT for at least 6 months prior to initiating migalastat therapy.

The term "ERT-experienced patient" refers to a Fabry patient that was receiving ERT immediately prior to initiating migalastat therapy. In some embodiments, the ERT-experienced patient has received at least 12 months of ERT immediately prior to initiating migalastat therapy.

As used herein, the term "free base equivalent" or "FBE" refers to the amount of migalastat present in the migalastat or salt thereof. In other words, the term "FBE" means either an amount of migalastat free base, or the equivalent amount of migalastat free base that is provided by a salt of migalastat. For example, due to the weight of the hydrochloride salt, 150 mg of migalastat hydrochloride only provides as much migalastat as 123 mg of the free base form of migalastat. Other salts are expected to have different conversion factors, depending on the molecular weight of the salt.

The term "migalastat" encompasses migalastat free base or a pharmaceutically acceptable salt thereof (e.g., migalastat HCl), unless specifically indicated to the contrary.

The terms "mutation" and "variant" (e.g., as in "amenable mutation or variant") refer to a change in the nucleotide sequence of a gene or a chromosome. The two terms referred herein are typically used together - *e.g.,* as in "mutation or variant"- referring to the change in nucleotide sequence stated in the previous sentence. If only one of the two terms is recited for some reason, the missing term was intended to be included and one should understand as such. Furthermore, the terms "amenable mutation" and "amenable variant" refer to a mutation or variant that is amenable to PC therapy, *e.g.,* a mutation that is amenable to migalastat therapy. A particular type of amenable mutation or variant is a "HEK assay amenable mutation or variant", which is a mutation or variant that is determined to be amenable to migalastat therapy according to the criteria in the *in vitro* HEK assay described herein and in U.S. Patent No. 8,592,362.

The terms "about" and "approximately" shall generally mean an acceptable degree of error for the quantity measured given the nature or precision of the measurements. Typical, exemplary degrees of error are within 20 percent (%), preferably within 10%, and more preferably within 5% of a given value or range of values. Alternatively, and particularly in biological systems, the terms "about" and "approximately" may mean values that are within an order of magnitude, preferably within 10- or 5-fold, and more preferably within 2-fold of a given value. Numerical quantities given herein are approximate unless stated otherwise, meaning that the term "about" or "approximately" can be inferred when not expressly stated.

### Fabry Disease

Fabry disease is a rare, progressive and devastating X-linked LSD. Mutations in the GLA gene result in a deficiency of the lysosomal enzyme, α-Gal A, which is required for glycosphingolipid metabolism. Beginning early in life, the reduction in α-Gal A activity results in an accumulation of glycosphingolipids, including GL-3 and plasma lyso-Gb3, and leads to the symptoms and life-limiting sequelae of Fabry disease, including pain, gastrointestinal symptoms, renal failure, cardiomyopathy, cerebrovascular events, and early mortality. Early initiation of therapy and lifelong treatment provide an opportunity to slow disease progression and prolong life expectancy.

Fabry disease encompasses a spectrum of disease severity and age of onset, although it has traditionally been divided into 2 main phenotypes, "classic" and "late-onset". The classic phenotype has been ascribed primarily to males with undetectable to low α-Gal A activity and earlier onset of renal, cardiac and/or cerebrovascular manifestations. The late-onset phenotype has been ascribed primarily to males with higher residual α-Gal A activity and later onset of these disease manifestations. Heterozygous female carriers typically express the late-onset phenotype but depending on the pattern of X-chromosome inactivation may also display the classic phenotype.

More than 1,000 Fabry disease-causing GLA mutations have been identified. Approximately 60% are missense mutations, resulting in single amino acid substitutions in the α-Gal A enzyme. Missense GLA mutations often result in the production of abnormally folded and unstable forms of α-Gal A and the majority are associated with the classic phenotype. Normal cellular quality control mechanisms in the ER block the transit of these abnormal proteins to lysosomes and target them for premature degradation and elimination. Many missense mutant forms are targets for migalastat, an α-Gal A-specific pharmacological chaperone.

The clinical manifestations of Fabry disease span a broad spectrum of severity and roughly correlate with a patient's residual α-Gal A levels. The majority of currently treated patients are referred to as classic Fabry patients, most of whom are males. These patients experience disease of various organs, including the kidneys, heart and brain, with disease symptoms first appearing in adolescence and typically progressing in severity until death in the fourth or fifth decade of life. A number of recent studies suggest that there are a large number of undiagnosed males and females that have a range of Fabry disease symptoms, such as impaired cardiac or renal function and strokes, that usually first appear in adulthood. Individuals with this type of Fabry disease, referred to as later-onset Fabry disease, tend to have higher residual α-Gal A levels than classic Fabry patients. Individuals with later-onset Fabry disease typically first experience disease symptoms in adulthood, and often have disease symptoms focused on a single organ, such as enlargement of the left ventricle or progressive kidney failure. In addition, later-onset Fabry disease may also present in the form of strokes of unknown cause.

Fabry patients have progressive kidney impairment, and untreated patients exhibit end-stage renal impairment by the fifth decade of life. Deficiency in α-Gal A activity leads to accumulation of GL-3 and related glycosphingolipids in many cell types including cells in the kidney. GL-3 accumulates in podocytes, epithelial cells and the tubular cells of the distal tubule and loop of Henle. Impairment in kidney function can manifest as proteinuria and reduced glomerular filtration rate.

Because Fabry disease is rare, involves multiple organs, has a wide age range of onset, and is heterogeneous, proper diagnosis is a challenge. Awareness is low among health care professionals and misdiagnoses are frequent. Diagnosis of Fabry disease is most often confirmed on the basis of decreased α-Gal A activity in plasma or peripheral leukocytes (WBCs) once a patient is symptomatic, coupled with mutational analysis. In females, diagnosis is even more challenging since the enzymatic identification of carrier females is less reliable due to random X-chromosomal inactivation in some cells of carriers. For example, some obligate carriers (daughters of classically affected males) have α-Gal A enzyme activities ranging from normal to very low activities. Since carriers can have normal α-Gal A enzyme activity in leukocytes, only the identification of an α-Gal A mutation by genetic testing provides precise carrier identification and/or diagnosis.

In one or more embodiments, mutant forms of α-Gal A are considered to be amenable to migalastat are defined as showing a relative increase (+10 µM migalastat) of ≥1.20-fold and an absolute increase (+ 10 µM migalastat) of ≥ 3.0% wild-type (WT) when the mutant form of α-Gal A is expressed in HEK-293 cells (referred to as the "HEK assay") according to Good Laboratory Practice (GLP)-validated *in vitro* assay (GLP HEK or Migalastat Amenability Assay). Such mutations are also referred to herein as "HEK assay amenable" mutations.

Previous screening methods have been provided that assess enzyme enhancement prior to the initiation of treatment. For example, an assay using HEK-293 cells has been utilized in clinical trials to predict whether a given mutation will be responsive to pharmacological chaperone (e.g., migalastat) treatment. In this assay, cDNA constructs are created. The corresponding α-Gal A mutant forms are transiently expressed in HEK-293 cells. Cells are then incubated ± migalastat (17 nM to 1 mM) for 4 to 5 days. After, α-Gal A levels are measured in cell lysates using a synthetic fluorogenic substrate (4-MU-α-Gal) or by western blot. This has been done for known disease-causing missense or small in-frame insertion/deletion mutations. Mutations that have previously been identified as responsive to a PC (e.g., migalastat) using these methods are listed in U.S. Patent No. 8,592,362.

### Pharmacological Chaperones

The binding of small molecule inhibitors of enzymes associated with LSDs can increase the stability of both mutant enzyme and the corresponding wild-type enzyme (see U.S. Pat. Nos. 6,274,597; 6,583,158; 6,589,964; 6,599,919; 6,916,829, and 7,141,582). In particular, administration of small molecule derivatives of glucose and galactose, which are specific, selective competitive inhibitors for several target lysosomal enzymes, effectively increased the stability of the enzymes in cells *in vitro* and, thus, increased trafficking of the enzymes to the lysosome. Thus, by increasing the amount of enzyme in the lysosome, hydrolysis of the enzyme substrates is expected to increase. The original theory behind this strategy was as follows: since the mutant enzyme protein is unstable in the ER (Ishii et al., Biochem. Biophys. Res. Comm. 1996; 220: 812-815), the enzyme protein is retarded in the normal transport pathway (ER-Golgi apparatus→endosomes→lysosome) and prematurely degraded. Therefore, a compound which binds to and increases the stability of a mutant enzyme, may serve as a "chaperone" for the enzyme and increase the amount that can exit the ER and move to the lysosomes. In addition, because the folding and trafficking of some wild-type proteins is incomplete, with up to 70% of some wild-type proteins being degraded in some instances prior to reaching their final cellular location, the chaperones can be used to stabilize wild-type enzymes and increase the amount of enzyme which can exit the ER and be trafficked to lysosomes.

The pharmacological chaperone of the present invention is migalastat or a salt thereof. The compound migalastat, also known as 1-deoxygalactonojirimycin (1-DGJ) or (2R,3S,4R,5S)-2-(hydroxymethyl) piperdine-3,4,5-triol is a compound having the following chemical formula:

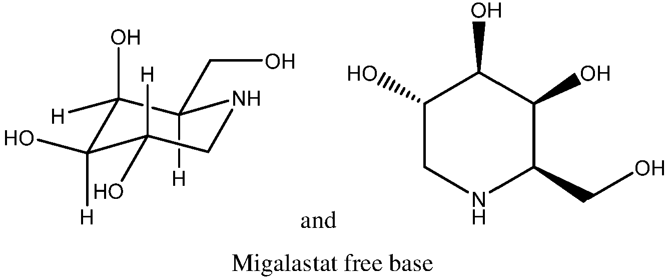

As discussed herein, pharmaceutically acceptable salts of migalastat may also be used in the present invention. When a salt of migalastat is used, the dosage of the salt will be adjusted so that the dose of migalastat received by the patient is equivalent to the amount which would have been received had the migalastat free base been used. One example of a pharmaceutically acceptable salt of migalastat is migalastat HCl:

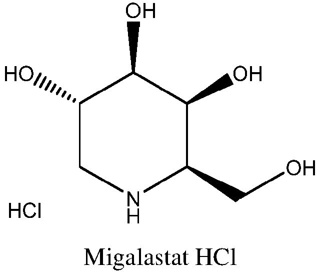

Migalastat is a low molecular weight iminosugar and is an analogue of the terminal galactose of GL-3. *In vitro* and *in vivo* pharmacologic studies have demonstrated that migalastat acts as a pharmacological chaperone, selectively and reversibly binding, with high affinity, to the active site of wild-type α-Gal A and specific mutant forms of α-Gal A, the genotypes of which are referred to as HEK assay amenable mutations. Migalastat binding stabilizes these mutant forms of α-Gal A in the endoplasmic reticulum facilitating their proper trafficking to lysosomes where dissociation of migalastat allows α-Gal A to reduce the level of GL-3 and other substrates. Approximately 30-50% of patients with Fabry disease have HEK assay amenable mutations; the majority of which are associated with the classic phenotype of the disease.

HEK assay amenable mutations include at least those mutations listed in a pharmacological reference table (*e.g.,* the ones recited in the U.S. or International Product labels for a migalastat product such as GALAFOLD^{®}). As used herein, "pharmacological reference table" refers to any publicly accessible written or electronic record, included in either the product label within the packaging of a migalastat product (e.g., GALAFOLD^{®}) or in a website accessible by health care providers, that conveys whether a particular mutation or variant is responsive to migalastat (e.g., GALAFOLD^{®}) PC therapy, and is not necessarily limited to written records presented in tabular form. In one embodiment of the present invention, a "pharmacological reference table" thus refers to any depository of information that includes one or more amenable mutations or variants. An exemplary pharmacological reference table for HEK assay amenable mutations can be found in the summary of product characteristics and/or prescribing information for GALAFOLD^{®} in various countries in which GALAFOLD^{®} is approved for use, or at a website such as www.galafoldamenabilitytable.com or www.fabrygenevariantsearch.com.

An exemplary pharmacological reference table for HEK assay amenable mutations is provided in Table 1 below. In one or more embodiments, if a double mutation is present on the same chromosome (males and females), that patient is considered HEK assay amenable if the double mutation is present in one entry in Table 1 (e.g., D55V/Q57L). In some embodiments, if a double mutation is present on different chromosomes (only in females) that patient is considered HEK assay amenable if either one of the individual mutations is present in Table 1.

**Table 1. HEK Assay Amenable Mutations**

| **Nucleotide change** | **Nucleotide change** | **Protein sequence change** |
|---|---|---|
| c.7C>G | c.C7G | L3V |
| c.8T>C | c.T8C | L3P |
| c.[11G>T; 620A>C] | c.G11T/A620C | R4M/Y207S |
| c.13A>G | c.A13G | N5D |
| c.15C>G | c.C15G | N5K |
| c.16C>A | c.C16A | P6T |
| c.16C>T | c.C16T | P6S |
| c.17C>A | c.C17A | P6Q |
| c.17C>G | c.C17G | P6R |
| c.17C>T | c.C 17T | P6L |
| c.19G>A | c.G19A | E7K |
| c.20A>T | c.A20T | E7V |
| c.21A>T | c.A21T | E7D |
| c.22C>A | c.C22A | L8I |
| c.23T>A | c.T23A | L8Q |
| c.23T>C | c.T23C | L8P |
| c.25C>T | c.C25T | H9Y |
| c.26A>G | c.A26G | H9R |
| c.26A>T | c.A26T | H9L |
| c.27T>A | c.T27A | H9Q |
| c.28C>A | c.C28A | L10M |
| c.28C>G | c.C28G | L10V |
| c.29T>A | c.T29A | L10Q |
| c.29T>C | c.T29C | L10P |
| c.29T>G | c.T29G | L10R |
| c.31G>A | c.G31A | G11S |
| c.31G>C | c.G31C | G11R |
| c.31G>T | c.G31T | G11C |
| c.32G>A | c.G32A | G11D |
| c.32G>T | c.G32T | G11V |
| c.34T>A | c.T34A | C12S |
| c.34T>C | c.T34C | C12R |
| c.34T>G | c.T34G | C12G |
| c.35G>A | c.G35A | C12Y |
| c.37G>A | c.G37A | A13T |
| c.37G>C | c.G37C | A13P |
| c.38C>A | c.C38A | A13E |
| c.38C>G | c.C38G | A13G |
| c.40C>G | c.C40G | L14V |
| c.40C>T | c.C40T | L14F |
| c.41T>A | c.T41A | L14H |
| c.43G>A | c.G43A | A15T |
| c.44C>G | c.C44G | A15G |
| c.49C>A | c.C49A | R17S |
| c.49C>G | c.C49G | R17G |
| c.49C>T | c.C49T | R17C |
| c.50G>A | c.G50A | R17H |
| c.50G>C | c.G50C | R17P |
| c.52T>A | c.T52A | F18I |
| c.53T>G | c.T53G | F18C |
| c.54C>G | c.C54G | F18L |
| c.58G>C | c.G58C | A20P |
| c.59C>A | c.C59A | A20D |
| c.59C>G | c.C59G | A20G |
| c.62T>A | c.T62A | L21H |
| c.64G>A | c.G64A | V22I |
| c.64G>C | c.G64C | V22L |
| c.64G>T | c.G64T | V22F |
| c.65T>C | c.T65C | V22A |
| c.65T>G | c.T65G | V22G |
| c.67T>A | c.T67A | S23T |
| c.67T>C | c.T67C | S23P |
| c.70T>C or c.70T>A | c.T70C or c.T70A | W24R |
| c.70T>G | c.T70G | W24G |
| c.71G>C | c.G71C | W24S |
| c.72G>C or c.72G>T | c.G72C or c.G72T | W24C |
| c.73G>C | c.G73C | D25H |
| c.77T>A | c.T77A | I26N |
| c.79C>A | c.C79A | P27T |
| c.79C>G | c.C79G | P27A |
| c.79C>T | c.C79T | P27S |
| c.80C>T | c.C80T | P27L |
| c.82G>C | c.G82C | G28R |
| c.82G>T | c.G82T | G28W |
| c.83G>A | c.G83A | G28E |
| c.85G>C | c.G85C | A29P |
| c.86C>A | c.C86A | A29D |
| c.86C>G | c.C86G | A29G |
| c.86C>T | c.C86T | A29V |
| c.88A>G | c.A88G | R30G |
| c.94C>A | c.C94A | L32M |
| c.94C>G | c.C94G | L32V |
| c.95T>A | c.T95A | L32Q |
| c.95T>C | c.T95C | L32P |
| c.95T>G | c.T95G | L32R |
| c.97G>C | c.G97C | D33H |
| c.97G>T | c.G97T | D33Y |
| c.98A>C | c.A98C | D33A |
| c.98A>G | c.A98G | D33G |
| c.98A>T | c.A98T | D33V |
| c.99C>G | c.C99G | D33E |
| c.100A>C | c.A100C | N34H |
| c.100A>G | c.A100G | N34D |
| c.101A>C | c.A101C | N34T |
| c.101A>G | c.A101G | N34S |
| c.102T>G or c.102T>A | c.T102G or c.T102A | N34K |
| c.103G>C or c.103G>A | c.G103C or c.G103A | G35R |
| c.104G>A | c.G104A | G35E |
| c.104G>C | c.G104C | G35A |
| c.104G>T | c.G104T | G35V |
| c.106T>A | c.T106A | L36M |
| c.106T>G | c.T106G | L36V |
| c.107T>C | c.T107C | L36S |
| c.107T>G | c.T107G | L36W |
| c.108G>C or c.108G>T | c.G108C or c.G108T | L36F |
| c.109G>A | c.G109A | A37T |
| c.109G>T | c.G109T | A37S |
| c.110C>A | c.C110A | A37E |
| c.110C>G | c.C110G | A37G |
| c.110C>T | c.C110T | A37V |
| c.112A>G | c.A112G | R38G |
| c.112A>T | c.A112T | R38W |
| c.113G>T | c.G113T | R38M |
| c.114G>C | c.G114C | R38S |
| c.115A>G | c.A115G | T39A |
| c.115A>T | c.A115T | T39S |
| c.116C>A | c.C116A | T39K |
| c.116C>G | c.C116G | T39R |
| c.116C>T | c.C116T | T39M |
| c.121A>G | c.A121G | T41A |
| c.122C>A | c.C122A | T41N |
| c.122C>G | c.C 122G | T41S |
| c.122C>T | c.C122T | T41I |
| c.124A>C or c.124A>T | c.A124C or c.A124T | M42L |
| c.124A>G | c.A124G | M42V |
| c.125T>A | c.T125A | M42K |
| c.125T>C | c.T125C | M42T |
| c.125T>G | c.T125G | M42R |
| c.126G>A or c.126G>C or c.126G>T | c.G126A or c.G126C or c.G126T | M42I |
| c.128G>C | c.G128C | G43A |
| c.133C>A | c.C133A | L45M |
| c.133C>G | c.C133G | L45V |
| c.136C>A | c.C136A | H46N |
| c.136C>G | c.C136G | H46D |
| c.137A>C | c.A137C | H46P |
| c.138C>G | c.C138G | H46Q |
| c.142G>C | c.G142C | E48Q |
| c.143A>C | c.A143C | E48A |
| c.149T>A | c.T149A | F50Y |
| c.151A>G | c.A151G | M51V |
| c.152T>A | c.T152A | M51K |
| c.152T>C | c.T152C | M51T |
| c.152T>G | c.T152G | M51R |
| c.153G>A or c.153G>T or c.153G>C | c.G153A or c.G153T or c.G153C | M51I |
| c.157A>C | c.A157C | N53H |
| c.[157A>C; 158A>T] | c.A157C/A158T | N53L |
| c.157A>G | c.A157G | N53D |
| c.157 A>T | c.A157T | N53Y |
| c.158A>C | c.A158C | N53T |
| c.158A>G | c.A158G | N53S |
| c.158A>T | c.A158T | N53I |
| c.159C>G or c.159C>A | c.C159G or c.C159A | N53K |
| c.160C>G | c.C160G | L54V |
| c.160C>T | c.C160T | L54F |
| c.161T>A | c.T161A | L54H |
| c.161T>C | c.T161C | L54P |
| c.161T>G | c.T161G | L54R |
| c.163G>C | c.G163C | D55H |
| c.163G>T | c.G163T | D55Y |
| c.164A>C | c.A164C | D55A |
| c.164A>G | c.A164G | D55G |
| c.164A>T | c.A164T | D55V |
| c.[164A>T; 170A>T] | c.A164T/A170T | D55V/Q57L |
| c.165C>G | c.C165G | D55E |
| c.167G>A | c.G167A | C56Y |
| c.167G>T | c.G167T | C56F |
| c.168C>G | c.C168G | C56W |
| c.170A>G | c.A170G | Q57R |
| c.170A>T | c.A170T | Q57L |
| c.172G>A | c.G172A | E58K |
| c.175G>A | c.G175A | E59K |
| c.175G>C | c.G175C | E59Q |
| c.176A>C | c.A176C | E59A |
| c.176A>G | c.A176G | E59G |
| c.176A>T | c.A176T | E59V |
| c.177G>C | c.G177C | E59D |
| c.178C>A | c.C178A | P60T |
| c.178C>G | c.C 178G | P60A |
| c.178C>T | c.C178T | P60S |
| c.179C>A | c.C179A | P60Q |
| c.179C>G | c.C 179G | P60R |
| c.179C>T | c.C179T | P60L |
| c.182A>T | c.A182T | D61V |
| c.183T>A | c.T183A | D61E |
| c.184_185insTAG | c.184_185insTAG | S62delinsLA |
| c.184T>C | c.T184C | S62P |
| c.184T>G | c.T184G | S62A |
| c.185C>A | c.C185A | S62Y |
| c.185C>G | c.C185G | S62C |
| c.185C>T | c.C185T | S62F |
| c.190A>C | c.A190C | I64L |
| c.190A>G | c.A190G | I64V |
| c.193A>G | c.A193G | S65G |
| c.193A>T | c.A193T | S65C |
| c.195T>A | c.T195A | S65R |
| c.196G>A | c.G196A | E66K |
| c.197A>G | c.A197G | E66G |
| c.197A>T | c.A197T | E66V |
| c.198G>C | c.G198C | E66D |
| c.199A>C | c.A199C | K67Q |
| c.199A>G | c.A199G | K67E |
| c.200A>C | c.A200C | K67T |
| c.200A>T | c.A200T | K67M |
| c.201G>C | c.G201C | K67N |
| c.202C>A | c.C202A | L68I |
| c.205T>A | c.T205A | F69I |
| c.206T>A | c.T206A | F69Y |
| c.207C>A or c.207C>G | c.C207A or c.C207G | F69L |
| c.208A>T | c.A208T | M70L |
| c.209T>A | c.T209A | M70K |
| c.209T>G | c.T209G | M70R |
| c.210G>C | c.G210C | M70I |
| c.211G>C | c.G211C | E71Q |
| c.212A>C | c.A212C | E71A |
| c.212A>G | c.A212G | E71G |
| c.212A>T | c.A212T | E71V |
| c.213G>C | c.G213C | E71D |
| c.214A>G | c.A214G | M72V |
| c.214A>T | c.A214T | M72L |
| c.215T>C | c.T215C | M72T |
| c.216G>A or c.216G>T or c.216G>C | c.G216A or c.G216T or c.G216C | M72I |
| c.217G>A | c.G217A | A73T |
| c.217G>T | c.G217T | A73S |
| c.218C>T | c.C218T | A73V |
| c.220G>A | c.G220A | E74K |
| c.221A>G | c.A221G | E74G |
| c.221A>T | c.A221T | E74V |
| c.222G>C | c.G222C | E74D |
| c.223C>T | c.C223T | L75F |
| c.224T>C | c.T224C | L75P |
| c.226A>G | c.A226G | M76V |
| c.227T>C | c.T227C | M76T |
| c.229G>A | c.G229A | V77I |
| c.229G>C | c.G229C | V77L |
| c.232T>C | c.T232C | S78P |
| c.233C>T | c.C233T | S78L |
| c.235G>A | c.G235A | E79K |
| c.235G>C | c.G235C | E79Q |
| c.236A>C | c.A236C | E79A |
| c.236A>G | c.A236G | E79G |
| c.236A>T | c.A236T | E79V |
| c.237A>T | c.A237T | E79D |
| c.238G>A | c.G238A | G80S |
| c.238G>T | c.G238T | G80C |
| c.239G>A | c.G239A | G80D |
| c.239G>C | c.G239C | G80A |
| c.239G>T | c.G239T | G80V |
| c.242G>T | c.G242T | W81L |
| c.244A>G | c.A244G | K82E |
| c.245A>C | c.A245C | K82T |
| c.245A>G | c.A245G | K82R |
| c.245A>T | c.A245T | K82M |
| c.246G>C | c.G246C | K82N |
| c.247G>A | c.G247A | D83N |
| c.248A>C | c.A248C | D83A |
| c.248A>G | c.A248G | D83G |
| c.248A>T | c.A248T | D83V |
| c.249T>A | c.T249A | D83E |
| c.250G>A | c.G250A | A84T |
| c.250G>C | c.G250C | A84P |
| c.250G>T | c.G250T | A84S |
| c.251C>A | c.C251A | A84E |
| c.251C>G | c.C251G | A84G |
| c.251C>T | c.C251T | A84V |
| c.253G>A | c.G253A | G85S |
| c.[253G>A; 254G>A] | c.G253A/G254A | G85N |
| c.[253G>A; 254G>T; 255T>G] | c.G253A/G254T/T255G | G85M |
| c.253G>C | c.G253C | G85R |
| c.253G>T | c.G253T | G85C |
| c.254G>A | c.G254A | G85D |
| c.254G>C | c.G254C | G85A |
| c.257A>T | c.A257T | Y86F |
| c.260A>G | c.A260G | E87G |
| c.261G>C or c.261G>T | c.G261C or c.G261T | E87D |
| c.262T>A | c.T262A | Y88N |
| c.262T>C | c.T262C | Y88H |
| c.263A>C | c.A263C | Y88S |
| c.263A>G | c.A263G | Y88C |
| c.265C>G | c.C265G | L89V |
| c.265C>T | c.C265T | L89F |
| c.271A>C | c.A271C | I91L |
| c.271A>T | c.A271T | I91F |
| c.272T>C | c.T272C | I91T |
| c.272T>G | c.T272G | I91S |
| c.273T>G | c.T273G | I91M |
| c.286A>G | c.A286G | M96V |
| c.286A>T | c.A286T | M96L |
| c.287T>C | c.T287C | M96T |
| c.288G>A or c.288G>T or c.288G>C | c.G288A or c.G288T or c.G288C | M96I |
| c.289G>A | c.G289A | A97T |
| c.289G>C | c.G289C | A97P |
| c.289G>T | c.G289T | A97S |
| c.290C>A | c.C290A | A97D |
| c.290C>T | c.C290T | A97V |
| c.293C>A | c.C293A | P98H |
| c.293C>G | c.C293G | P98R |
| c.293C>T | c.C293T | P98L |
| c.295C>G | c.C295G | Q99E |
| c.296A>C | c.A296C | Q99P |
| c.296A>G | c.A296G | Q99R |
| c.296A>T | c.A296T | Q99L |
| c.301G>C | c.G301C | D101H |
| c.302A>C | c.A302C | D101A |
| c.302A>G | c.A302G | D101G |
| c.302A>T | c.A302T | D101V |
| c.303T>A | c.T303A | D101E |
| c.304T>A | c.T304A | S102T |
| c.304T>C | c.T304C | S102P |
| c.304T>G | c.T304G | S102A |
| c.305C>T | c.C305T | S102L |
| c.310G>A | c.G310A | G104S |
| c.311G>A | c.G311A | G104D |
| c.311G>C | c.G311C | G104A |
| c.311G>T | c.G311T | G104V |
| c.313A>G | c.A313G | R105G |
| c.314G>A | c.G314A | R105K |
| c.314G>C | c.G314C | R105T |
| c.314G>T | c.G314T | R105I |
| c.316C>A | c.C316A | L106I |
| c.316C>G | c.C316G | L106V |
| c.316C>T | c.C316T | L106F |
| c.317T>A | c.T317A | L106H |
| c.317T>C | c.T317C | L106P |
| c.319C>A | c.C319A | Q107K |
| c.319C>G | c.C319G | Q107E |
| c.320A>G | c.A320G | Q107R |
| c.321G>C | c.G321C | Q107H |
| c.322G>A | c.G322A | A108T |
| c.323C>A | c.C323A | A108E |
| c.323C>T | c.C323T | A108V |
| c.325G>A | c.G325A | D109N |
| c.325G>C | c.G325C | D109H |
| c.325G>T | c.G325T | D109Y |
| c.326A>C | c.A326C | D109A |
| c.326A>G | c.A326G | D109G |
| c.327C>G | c.C327G | D109E |
| c.328C>A | c.C328A | P110T |
| c.334C>G | c.C334G | R112G |
| c.335G>A | c.G335A | R112H |
| c.335G>T | c.G335T | R112L |
| c.337T>A | c.T337A | F113I |
| c.337T>C or c.339T>A or c.339T>G | c.T337C or c.T339A or c.T339G | F113L |
| c.337T>G | c.T337G | F113V |
| c.338T>A | c.T338A | F113Y |
| c.341C>T | c.C341T | P114L |
| c.343C>A | c.C343A | H115N |
| c.343C>G | c.C343G | H115D |
| c.346G>C | c.G346C | G116R |
| c.350T>C | c.T350C | I117T |
| c.351T>G | c.T351G | I117M |
| c.352C>T | c.C352T | R118C |
| c.361G>A | c.G361A | A121T |
| c.362C>T | c.C362T | A121V |
| c.367T>A | c.T367A | Y123N |
| c.367T>G | c.T367G | Y123D |
| c.368A>C | c.A368C | Y123S |
| c.368A>G | c.A368G | Y123C |
| c.368A>T | c.A368T | Y123F |
| c.370G>A | c.G370A | V124I |
| c.371T>G | c.T371G | V124G |
| c.373C>A | c.C373A | H125N |
| c.373C>G | c.C373G | H125D |
| c.373C>T | c.C373T | H125Y |
| c.374A>G | c.A374G | H125R |
| c.374A>T | c.A374T | H125L |
| c.376A>G | c.A376G | S126G |
| c.376A>T | c.A376T | S126C |
| c.377G>T | c.G377T | S126I |
| c.379A>G | c.A379G | K127E |
| c.383G>A | c.G383A | G128E |
| c.383G>C | c.G383C | G128A |
| c.385C>G | c.C385G | L129V |
| c.388A>C | c.A388C | K130Q |
| c.389A>T | c.A389T | K130M |
| c.390G>C | c.G390C | K130N |
| c.391C>G | c.C391G | L131V |
| c.397A>C | c.A397C | I133L |
| c.397A>G | c.A397G | I133V |
| c.397A>T | c.A397T | I133F |
| c.398T>C | c.T398C | I133T |
| c.399T>G | c.T399G | I133M |
| c.[399T>G; 434T>C] | c.T399G/T434C | I133M/F145S |
| c.403G>A | c.G403A | A135T |
| c.403G>T | c.G403T | A135S |
| c.404C>A | c.C404A | A135E |
| c.404C>G | c.C404G | A135G |
| c.404C>T | c.C404T | A135V |
| c.406G>A | c.G406A | D136N |
| c.407A>C | c.A407C | D136A |
| c.407A>T | c.A407T | D136V |
| c.408T>A or c.408T>G | c.T408A or c.T408G | D136E |
| c.409G>A | c.G409A | V137I |
| c.409G>C | c.G409C | V137L |
| c.410T>A | c.T410A | V137D |
| c.410T>C | c.T410C | V137A |
| c.410T>G | c.T410G | V137G |
| c.413G>C | c.G413C | G138A |
| c.415A>C | c.A415C | N139H |
| c.415A>T | c.A415T | N139Y |
| c.416A>G | c.A416G | N139S |
| c.416A>T | c.A416T | N139I |
| c.417T>A | c.T417A | N139K |
| c.418A>C | c.A418C | K140Q |
| c.418A>G | c.A418G | K140E |
| c.419A>C | c.A419C | K140T |
| c.419A>G | c.A419G | K140R |
| c.419A>T | c.A419T | K140I |
| c.420A>T | c.A420T | K140N |
| c.421A>T | c.A421T | T141S |
| c.427G>A | c.G427A | A143T |
| c.428C>A | c.C428A | A143E |
| c.428C>G | c.C428G | A143G |
| c.428C>T | c.C428T | A143V |
| c.430G>A | c.G430A | G144S |
| c.430G>C | c.G430C | G144R |
| c.430G>T | c.G430T | G144C |
| c.431G>A | c.G431A | G144D |
| c.431G>C | c.G431C | G144A |
| c.431G>T | c.G431T | G144V |
| c.433T>G | c.T433G | F145V |
| c.434T>A | c.T434A | F145Y |
| c.434T>C | c.T434C | F145S |
| c.434T>G | c.T434G | F145C |
| c.435C>G | c.C435G | F145L |
| c.436C>A | c.C436A | P146T |
| c.436C>G | c.C436G | P146A |
| c.436C>T | c.C436T | P146S |
| c.437C>A | c.C437A | P146H |
| c.437C>G | c.C437G | P146R |
| c.437C>T | c.C437T | P146L |
| c.440G>C | c.G440C | G147A |
| c.442A>G | c.A442G | S148G |
| c.442A>T | c.A442T | S148C |
| c.443G>C | c.G443C | S148T |
| c.446T>G | c.T446G | F149C |
| c.449G>A | c.G449A | G150E |
| c.449G>T | c.G449T | G150V |
| c.451T>G | c.T451G | Y151D |
| c.452A>C | c.A452C | Y151S |
| c.452A>G | c.A452G | Y151C |
| c.454T>A | c.T454A | Y152N |
| c.454T>C | c.T454C | Y152H |
| c.454T>G | c.T454G | Y152D |
| c.455A>C | c.A455C | Y152S |
| c.455A>G | c.A455G | Y152C |
| c.455A>T | c.A455T | Y152F |
| c.457G>A | c.G457A | D153N |
| c.457G>C | c.G457C | D153H |
| c.457G>T | c.G457T | D153Y |
| c.458A>C | c.A458C | D153A |
| c.458A>T | c.A458T | D153V |
| c.465T>A or c.465T>G | c.T465A or c.T465G | D155E |
| c.466G>A | c.G466A | A156T |
| c.466G>T | c.G466T | A156S |
| c.467C>G | c.C467G | A156G |
| c.467C>T | c.C467T | A156V |
| c.469C>A | c.C469A | Q157K |
| c.469C>G | c.C469G | Q157E |
| c.470A>C | c.A470C | Q157P |
| c.470A>T | c.A470T | Q157L |
| c.471G>C or c.471G>T | c.G471C or c.G471T | Q157H |
| c.472A>G | c.A472G | T158A |
| c.472A>T | c.A472T | T158S |
| c.473C>A | c.C473A | T158N |
| c.473C>T | c.C473T | T158I |
| c.475T>A | c.T475A | F159I |
| c.475T>G | c.T475G | F159V |
| c.476T>A | c.T476A | F159Y |
| c.476T>G | c.T476G | F159C |
| c.477T>A | c.T477A | F159L |
| c.478G>A | c.G478A | A160T |
| c.478G>T | c.G478T | A160S |
| c.479C>A | c.C479A | A160D |
| c.479C>G | c.C479G | A160G |
| c.479C>T | c.C479T | A160V |
| c.481G>A | c.G481A | D161N |
| c.481G>C | c.G481C | D161H |
| c.481G>T | c.G481T | D161Y |
| c.482A>T | c.A482T | D161V |
| c.484T>G | c.T484G | W162G |
| c.485G>C | c.G485C | W162S |
| c.490G>A | c.G490A | V164I |
| c.490G>T | c.G490T | V164L |
| c.491T>C | c.T491C | V164A |
| c.493G>A | c.G493A | D165N |
| c.493G>C | c.G493C | D165H |
| c.494A>C | c.A494C | D165A |
| c.494A>G | c.A494G | D165G |
| c.495T>A | c.T495A | D165E |
| c.496_497delinsTC | c.496_497delinsTC | L166S |
| c.496C>A | c.C496A | L166M |
| c.496C>G | c.C496G | L166V |
| c.[496C>G; 497T>G] | c.C496G/T497G | L166G |
| c.497T>A | c.T497A | L166Q |
| c.499C>A | c.C499A | L167I |
| c.499C>G | c.C499G | L167V |
| c.505T>A | c.T505A | F169I |
| c.505T>G | c.T505G | F169V |
| c.506T>A | c.T506A | F169Y |
| c.506T>C | c.T506C | F169S |
| c.506T>G | c.T506G | F169C |
| c.507T>A | c.T507A | F169L |
| c.511G>A | c.G511A | G171S |
| c.512G>C | c.G512C | G171A |
| c.512G>T | c.G512T | G171V |
| c.517T>C | c.T517C | Y173H |
| c.518A>C | c.A518C | Y173S |
| c.518A>G | c.A518G | Y173C |
| c.518A>T | c.A518T | Y173F |
| c.520T>C | c.T520C | C174R |
| c.520T>G | c.T520G | C174G |
| c.523G>C | c.G523C | D175H |
| c.523G>T | c.G523T | D175Y |
| c.524A>G | c.A524G | D175G |
| c.524A>T | c.A524T | D175V |
| c.525C>G or c.525C>A | c.C525G or c.C525A | D175E |
| c.526A>T | c.A526T | S176C |
| c.528T>A | c.T528A | S176R |
| c.529T>A | c.T529A | L177M |
| c.529T>G | c.T529G | L177V |
| c.530T>C | c.T530C | L177S |
| c.530T>G | c.T530G | L177W |
| c.531G>C | c.G531C | L177F |
| c.532G>A | c.G532A | E178K |
| c.532G>C | c.G532C | E178Q |
| c.533A>C | c.A533C | E178A |
| c.533A>G | c.A533G | E178G |
| c.538T>A | c.T538A | L180M |
| c.538T>G | c.T538G | L180V |
| c.539T>C | c.T539C | L180S |
| c.539T>G | c.T539G | L180W |
| c.540G>C or c.540G>T | c.G540C or c.G540T | L180F |
| c.541G>A | c.G541A | A181T |
| c.541G>C | c.G541C | A181P |
| c.542C>T | c.C542T | A181V |
| c.544G>T | c.G544T | D182Y |
| c.545A>C | c.A545C | D182A |
| c.545A>G | c.A545G | D182G |
| c.545A>T | c.A545T | D182V |
| c.546T>A | c.T546A | D182E |
| c.548G>A | c.G548A | G183D |
| c.548G>C | c.G548C | G183A |
| c.550T>A | c.T550A | Y184N |
| c.550T>C | c.T550C | Y184H |
| c.551A>C | c.A551C | Y184S |
| c.551A>G | c.A551G | Y184C |
| c.551A>T | c.A551T | Y184F |
| c.553A>C | c.A553C | K185Q |
| c.553A>G | c.A553G | K185E |
| c.554A>C | c.A554C | K185T |
| c.554A>T | c.A554T | K185M |
| c.555G>C | c.G555C | K185N |
| c.556C>A | c.C556A | H186N |
| c.556C>G | c.C556G | H186D |
| c.556C>T | c.C556T | H186Y |
| c.557A>T | c.A557T | H186L |
| c.558C>G | c.C558G | H186Q |
| c.559_564dup | c.559_564dup | p.M187_S188dup |
| c.559A>T | c.A559T | M187L |
| c.559A>G | c.A559G | M187V |
| c.560T>C | c.T560C | M187T |
| c.561G>T or c.561G>A or c.561G>C | c.G561T or c.G561A or c.G561C | M187I |
| c.562T>A | c.T562A | S188T |
| c.562T>C | c.T562C | S188P |
| c.562T>G | c.T562G | S188A |
| c.563C>A | c.C563A | S188Y |
| c.563C>G | c.C563G | S188C |
| c.563C>T | c.C563T | S188F |
| c.565T>G | c.T565G | L189V |
| c.566T>C | c.T566C | L189S |
| c.567G>C or c.567G>T | c.G567C or c.G567T | L189F |
| c.568G>A | c.G568A | A190T |
| c.568G>T | c.G568T | A190S |
| c.569C>A | c.C569A | A190D |
| c.569C>G | c.C569G | A190G |
| c.569C>T | c.C569T | A190V |
| c.571C>A | c.C571A | L191M |
| c.571C>G | c.C571G | L191V |
| c.572T>A | c.T572A | L191Q |
| c.574A>C | c.A574C | N192H |
| c.574A>G | c.A574G | N192D |
| c.575A>C | c.A575C | N192T |
| c.575A>G | c.A575G | N192S |
| c.576T>A | c.T576A | N192K |
| c.577A>G | c.A577G | R193G |
| c.577A>T | c.A577T | R193W |
| c.578G>C | c.G578C | R193T |
| c.578G>T | c.G578T | R193M |
| c.580A>C | c.A580C | T194P |
| c.580A>G | c.A580G | T194A |
| c.580A>T or c.581C>G | c.A580T or c.C581G | T194S |
| c.581C>A | c.C581A | T194N |
| c.581C>T | c.C581T | T194I |
| c.583G>A | c.G583A | G195S |
| c.583G>C | c.G583C | G195R |
| c.583G>T | c.G583T | G195C |
| c.584G>T | c.G584T | G195V |
| c.586A>G | c.A586G | R196G |
| c.587G>A | c.G587A | R196K |
| c.587G>C | c.G587C | R196T |
| c.587G>T | c.G587T | R196I |
| c.589A>G | c.A589G | S197G |
| c.589A>T | c.A589T | S197C |
| c.590G>A | c.G590A | S197N |
| c.590G>C | c.G590C | S197T |
| c.590G>T | c.G590T | S197I |
| c.593T>C | c.T593C | I198T |
| c.593T>G | c.T593G | I198S |
| c.594T>G | c.T594G | I198M |
| c.595G>A | c.G595A | V199M |
| c.595G>C | c.G595C | V199L |
| c.596T>A | c.T596A | V199E |
| c.596T>C | c.T596C | V199A |
| c.596T>G | c.T596G | V199G |
| c.598T>A | c.T598A | Y200N |
| c.599A>C | c.A599C | Y200S |
| c.599A>G | c.A599G | Y200C |
| c.601T>A | c.T601A | S201T |
| c.601T>G | c.T601G | S201A |
| c.602C>A | c.C602A | S201Y |
| c.602C>G | c.C602G | S201C |
| c.602C>T | c.C602T | S201F |
| c.607G>C | c.G607C | E203Q |
| c.608A>C | c.A608C | E203A |
| c.608A>G | c.A608G | E203G |
| c.608A>T | c.A608T | E203V |
| c.609G>C or c.609G>T | c.G609C or c.G609T | E203D |
| c.610T>G | c.T610G | W204G |
| c.611G>C | c.G611C | W204S |
| c.611G>T | c.G611T | W204L |
| c.613C>A | c.C613A | P205T |
| c.613C>T | c.C613T | P205S |
| c.614C>T | c.C614T | P205L |
| c.616C>A | c.C616A | L206I |
| c.616C>G | c.C616G | L206V |
| c.616C>T | c.C616T | L206F |
| c.617T>A | c.T617A | L206H |
| c.617T>G | c.T617G | L206R |
| c.619T>C | c.T619C | Y207H |
| c.620A>C | c.A620C | Y207S |
| c.620A>T | c.A620T | Y207F |
| c.623T>A | c.T623A | M208K |
| c.623T>G | c.T623G | M208R |
| c.625T>A | c.T625A | W209R |
| c.625T>G | c.T625G | W209G |
| c.627G>C | c.G627C | W209C |
| c.628C>A | c.C628A | P210T |
| c.628C>T | c.C628T | P210S |
| c.629C>A | c.C629A | P210H |
| c.629C>T | c.C629T | P210L |
| c.631T>C | c.T631C | F211L |
| c.631T>G | c.T631G | F211V |
| c.632T>A | c.T632A | F211Y |
| c.632T>C | c.T632C | F211S |
| c.632T>G | c.T632G | F211C |
| c.635A>C | c.A635C | Q212P |
| c.636A>T | c.A636T | Q212H |
| c.637A>C | c.A637C | K213Q |
| c.637A>G | c.A637G | K213E |
| c.638A>G | c.A638G | K213R |
| c.638A>T | c.A638T | K213M |
| c.640C>A | c.C640A | P214T |
| c.640C>G | c.C640G | P214A |
| c.640C>T | c.C640T | P214S |
| c.641C>A | c.C641A | P214H |
| c.641C>G | c.C641G | P214R |
| c.641C>T | c.C641T | P214L |
| c.643A>C | c.A643C | N215H |
| c.643A>G | c.A643G | N215D |
| c.643A>T | c.A643T | N215Y |
| c.644A>C | c.A644C | N215T |
| c.644A>G | c.A644G | N215S |
| c.[644A>G; 937G>T] | c.A644G/G937T | N215S/D313Y |
| c.644A>T | c.A644T | N215I |
| c.645T>A | c.T645A | N215K |
| c.646T>A | c.T646A | Y216N |
| c.646T>C | c.T646C | Y216H |
| c.646T>G | c.T646G | Y216D |
| c.647A>C | c.A647C | Y216S |
| c.647A>G | c.A647G | Y216C |
| c.647A>T | c.A647T | Y216F |
| c.649A>C | c.A649C | T217P |
| c.649A>G | c.A649G | T217A |
| c.649A>T | c.A649T | T217S |
| c.650C>A | c.C650A | T217K |
| c.650C>G | c.C650G | T217R |
| c.650C>T | c.C650T | T217I |
| c.652G>A | c.G652A | E218K |
| c.652G>C | c.G652C | E218Q |
| c.653A>C | c.A653C | E218A |
| c.653A>G | c.A653G | E218G |
| c.653A>T | c.A653T | E218V |
| c.654A>T | c.A654T | E218D |
| c.655A>C | c.A655C | I219L |
| c.655A>T | c.A655T | I219F |
| c.656T>A | c.T656A | I219N |
| c.656T>C | c.T656C | I219T |
| c.656T>G | c.T656G | I219S |
| c.657C>G | c.C657G | I219M |
| c.659G>A | c.G659A | R220Q |
| c.659G>C | c.G659C | R220P |
| c.659G>T | c.G659T | R220L |
| c.661C>A | c.C661A | Q221K |
| c.661C>G | c.C661G | Q221E |
| c.662A>C | c.A662C | Q221P |
| c.662A>G | c.A662G | Q221R |
| c.662A>T | c.A662T | Q221L |
| c.663G>C | c.G663C | Q221H |
| c.664T>A | c.T664A | Y222N |
| c.664T>C | c.T664C | Y222H |
| c.664T>G | c.T664G | Y222D |
| c.665A>C | c.A665C | Y222S |
| c.665A>G | c.A665G | Y222C |
| c.670A>C | c.A670C | N224H |
| c.671A>C | c.A671C | N224T |
| c.671A>G | c.A671G | N224S |
| c.673C>G | c.C673G | H225D |
| c.679C>G | c.C679G | R227G |
| c.682A>C | c.A682C | N228H |
| c.682A>G | c.A682G | N228D |
| c.683A>C | c.A683C | N228T |
| c.683A>G | c.A683G | N228S |
| c.683A>T | c.A683T | N228I |
| c.685T>A | c.T685A | F229I |
| c.686T>A | c.T686A | F229Y |
| c.686T>C | c.T686C | F229S |
| c.687T>A or c.687T>G | c.T687A or c.T687G | F229L |
| c.688G>C | c.G688C | A230P |
| c.689C>A | c.C689A | A230D |
| c.689C>G | c.C689G | A230G |
| c.689C>T | c.C689T | A230V |
| c.694A>C | c.A694C | I232L |
| c.694A>G | c.A694G | I232V |
| c.695T>C | c.T695C | I232T |
| c.696T>G | c.T696G | I232M |
| c.698A>C | c.A698C | D233A |
| c.698A>G | c.A698G | D233G |
| c.698A>T | c.A698T | D233V |
| c.699T>A | c.T699A | D233E |
| c.703T>A | c.T703A | S235T |
| c.703T>G | c.T703G | S235A |
| c.710A>T | c.A710T | K237I |
| c.712A>G | c.A712G | S238G |
| c.712A>T | c.A712T | S238C |
| c.713G>A | c.G713A | S238N |
| c.713G>C | c.G713C | S238T |
| c.713G>T | c.G713T | S238I |
| c.715A>T | c.A715T | I239L |
| c.716T>C | c.T716C | I239T |
| c.717A>G | c.A717G | I239M |
| c.718A>G | c.A718G | K240E |
| c.719A>G | c.A719G | K240R |
| c.719A>T | c.A719T | K240M |
| c.720G>C or c.720G>T | c.G720C or c.G720T | K240N |
| c.721A>T | c.A721T | S241C |
| c.722G>C | c.G722C | S241T |
| c.722G>T | c.G722T | S241I |
| c.724A>C | c.A724C | I242L |
| c.724A>G | c.A724G | I242V |
| c.724A>T | c.A724T | I242F |
| c.725T>A | c.T725A | I242N |
| c.725T>C | c.T725C | I242T |
| c.725T>G | c.T725G | I242S |
| c.726C>G | c.C726G | I242M |
| c.727T>A | c.T727A | L243M |
| c.727T>G | c.T727G | L243V |
| c.728T>C | c.T728C | L243S |
| c.728T>G | c.T728G | L243W |
| c.729G>C or c.729G>T | c.G729C or c.G729T | L243F |
| c.730G>A | c.G730A | D244N |
| c.730G>C | c.G730C | D244H |
| c.730G>T | c.G730T | D244Y |
| c.731A>C | c.A731C | D244A |
| c.731A>G | c.A731G | D244G |
| c.731A>T | c.A731T | D244V |
| c.732C>G | c.C732G | D244E |
| c.733T>G | c.T733G | W245G |
| c.735G>C | c.G735C | W245C |
| c.736A>G | c.A736G | T246A |
| c.737C>A | c.C737A | T246K |
| c.737C>G | c.C737G | T246R |
| c.737C>T | c.C737T | T246I |
| c.739T>A | c.T739A | S247T |
| c.739T>G | c.T739G | S247A |
| c.740C>A | c.C740A | S247Y |
| c.740C>G | c.C740G | S247C |
| c.740C>T | c.C740T | S247F |
| c.742T>G | c.T742G | F248V |
| c.743T>A | c.T743A | F248Y |
| c.743T>G | c.T743G | F248C |
| c.744T>A | c.T744A | F248L |
| c.745A>C | c.A745C | N249H |
| c.745A>G | c.A745G | N249D |
| c.745A>T | c.A745T | N249Y |
| c.746A>C | c.A746C | N249T |
| c.746A>G | c.A746G | N249S |
| c.746A>T | c.A746T | N249I |
| c.747C>G or c.747C>A | c.C747G or c.C747A | N249K |
| c.748C>A | c.C748A | Q250K |
| c.748C>G | c.C748G | Q250E |
| c.749A>C | c.A749C | Q250P |
| c.749A>G | c.A749G | Q250R |
| c.749A>T | c.A749T | Q250L |
| c.750G>C | c.G750C | Q250H |
| c.751G>A | c.G751A | E251K |
| c.751G>C | c.G751C | E251Q |
| c.752A>G | c.A752G | E251G |
| c.752A>T | c.A752T | E251V |
| c.754A>G | c.A754G | R252G |
| c.757A>G | c.A757G | I253V |
| c.757A>T | c.A757T | I253F |
| c.758T>A | c.T758A | I253N |
| c.758T>C | c.T758C | I253T |
| c.758T>G | c.T758G | I253S |
| c.760-762delGTT or c.761-763del | c.760_762delGTT or c.761_763del | p.V254del |
| c.760G>T | c.G760T | V254F |
| c.761T>A | c.T761A | V254D |
| c.761T>C | c.T761C | V254A |
| c.761T>G | c.T761G | V254G |
| c.763G>A | c.G763A | D255N |
| c.763G>C | c.G763C | D255H |
| c.763G>T | c.G763T | D255Y |
| c.764A>C | c.A764C | D255A |
| c.764A>T | c.A764T | D255V |
| c.765T>A | c.T765A | D255E |
| c.766G>C | c.G766C | V256L |
| c.767T>A | c.T767A | V256D |
| c.767T>G | c.T767G | V256G |
| c.769G>A | c.G769A | A257T |
| c.769G>C | c.G769C | A257P |
| c.769G>T | c.G769T | A257S |
| c.770C>G | c.C770G | A257G |
| c.770C>T | c.C770T | A257V |
| c.772G>C or c.772G>A | c.G772C or c.G772A | G258R |
| c.773G>A | c.G773A | G258E |
| c.773G>T | c.G773T | G258V |
| c.775C>A | c.C775A | P259T |
| c.775C>G | c.C775G | P259A |
| c.775C>T | c.C775T | P259S |
| c.776C>A | c.C776A | P259Q |
| c.776C>G | c.C776G | P259R |
| c.776C>T | c.C776T | P259L |
| c.778G>T | c.G778T | G260W |
| c.779G>A | c.G779A | G260E |
| c.779G>C | c.G779C | G260A |
| c.781G>A | c.G781A | G261S |
| c.781G>C | c.G781C | G261R |
| c.781G>T | c.G781T | G261C |
| c.782G>C | c.G782C | G261A |
| c.787A>C | c.A787C | N263H |
| c.788A>C | c.A788C | N263T |
| c.788A>G | c.A788G | N263S |
| c.790G>A | c.G790A | D264N |
| c.790G>C | c.G790C | D264H |
| c.790G>T | c.G790T | D264Y |
| c.793C>G | c.C793G | P265A |
| c.794C>A | c.C794A | P265Q |
| c.794C>T | c.C794T | P265L |
| c.799A>G | c.A799G | M267V |
| c.799A>T | c.A799T | M267L |
| c.800T>C | c.T800C | M267T |
| c.802T>A | c.T802A | L268I |
| c.804A>T | c.A804T | L268F |
| c.805G>A | c.G805A | V269M |
| c.805G>C | c.G805C | V269L |
| c.806T>C | c.T806C | V269A |
| c.808A>C | c.A808C | I270L |
| c.808A>G | c.A808G | I270V |
| c.809T>C | c.T809C | I270T |
| c.809T>G | c.T809G | I270S |
| c.810T>G | c.T810G | I270M |
| c.811G>A | c.G811A | G271S |
| c.[811G>A; 937G>T] | c.G811A/G937T | G271S/D313Y |
| c.812G>A | c.G812A | G271D |
| c.812G>C | c.G812C | G271A |
| c.814A>G | c.A814G | N272D |
| c.818T>A | c.T818A | F273Y |
| c.823C>A | c.C823A | L275I |
| c.823C>G | c.C823G | L275V |
| c.827G>A | c.G827A | S276N |
| c.827G>C | c.G827C | S276T |
| c.829T>G | c.T829G | W277G |
| c.830G>T | c.G830T | W277L |
| c.831G>T or c.831G>C | c.G831T or c.G831C | W277C |
| c.832A>T | c.A832T | N278Y |
| c.833A>T | c.A833T | N278I |
| c.835C>G | c.C835G | Q279E |
| c.838C>A | c.C838A | Q280K |
| c.839A>G | c.A839G | Q280R |
| c.839A>T | c.A839T | Q280L |
| c.840A>T or c.840A>C | c.A840T or c.A840C | Q280H |
| c.841G>C | c.G841C | V281L |
| c.842T>A | c.T842A | V281E |
| c.842T>C | c.T842C | V281A |
| c.842T>G | c.T842G | V281G |
| c.844A>G | c.A844G | T282A |
| c.844A>T | c.A844T | T282S |
| c.845C>T | c.C845T | T282I |
| c.847C>G | c.C847G | Q283E |
| c.848A>T | c.A848T | Q283L |
| c.849G>C | c.G849C | Q283H |
| c.850A>G | c.A850G | M284V |
| c.850A>T | c.A850T | M284L |
| c.851T>C | c.T851C | M284T |
| c.852G>C | c.G852C | M284I |
| c.853G>A | c.G853A | A285T |
| c.854C>G | c.C854G | A285G |
| c.854C>T | c.C854T | A285V |
| c.856C>G | c.C856G | L286V |
| c.856C>T | c.C856T | L286F |
| c.857T>A | c.T857A | L286H |
| c.860G>T | c.G860T | W287L |
| c.862G>C | c.G862C | A288P |
| c.862G>T | c.G862T | A288S |
| c.863C>G | c.C863G | A288G |
| c.863C>T | c.C863T | A288V |
| c.865A>C | c.A865C | I289L |
| c.865A>G | c.A865G | I289V |
| c.866T>C | c.T866C | I289T |
| c.866T>G | c.T866G | I289S |
| c.868A>C or c.868A>T | c.A868C or c.A868T | M290L |
| c.868A>G | c.A868G | M290V |
| c.869T>C | c.T869C | M290T |
| c.870G>A or c.870G>C or c.870G>T | c.G870A or c.G870C or c.G870T | M290I |
| c.871G>A | c.G871A | A291T |
| c.871G>T | c.G871T | A291S |
| c.872C>G | c.C872G | A291G |
| c.874G>T | c.G874T | A292S |
| c.875C>G | c.C875G | A292G |
| c.877C>A | c.C877A | P293T |
| c.880T>A | c.T880A | L294I |
| c.880T>G | c.T880G | L294V |
| c.881T>C | c.T881C | L294S |
| c.882A>T | c.A882T | L294F |
| c.883T>A | c.T883A | F295I |
| c.883T>G | c.T883G | F295V |
| c.884T>A | c.T884A | F295Y |
| c.884T>C | c.T884C | F295S |
| c.884T>G | c.T884G | F295C |
| c.886A>G | c.A886G | M296V |
| c.886A>T or c.886A>C | c.A886T or c.A886C | M296L |
| c.887T>C | c.T887C | M296T |
| c.888G>A or c.888G>T or c.888G>C | c.G888A or c.G888T or c.G888C | M296I |
| c.889T>A | c.T889A | S297T |
| c.892A>G | c.A892G | N298D |
| c.893A>C | c.A893C | N298T |
| c.893A>G | c.A893G | N298S |
| c.893A>T | c.A893T | N298I |
| c.895G>A | c.G895A | D299N |
| c.895G>C | c.G895C | D299H |
| c.897C>G or c.897C>A | c.C897G or c.C897A | D299E |
| c.898C>A | c.C898A | L300I |
| c.898C>G | c.C898G | L300V |
| c.898C>T | c.C898T | L300F |
| c.899T>C | c.T899C | L300P |
| c.901C>G | c.C901G | R301G |
| c.902G>A | c.G902A | R301Q |
| c.902G>C | c.G902C | R301P |
| c.902G>T | c.G902T | R301L |
| c.904C>A | c.C904A | H302N |
| c.904C>G | c.C904G | H302D |
| c.904C>T | c.C904T | H302Y |
| c.905A>T | c.A905T | H302L |
| c.907A>G | c.A907G | I303V |
| c.907A>T | c.A907T | I303F |
| c.908T>A | c.T908A | I303N |
| c.908T>C | c.T908C | I303T |
| c.908T>G | c.T908G | I303S |
| c.911G>A | c.G911A | S304N |
| c.911G>C | c.G911C | S304T |
| c.911G>T | c.G911T | S304I |
| c.916C>G | c.C916G | Q306E |
| c.917A>C | c.A917C | Q306P |
| c.917A>T | c.A917T | Q306L |
| c.919G>A | c.G919A | A307T |
| c.919G>C | c.G919C | A307P |
| c.919G>T | c.G919T | A307S |
| c.920C>A | c.C920A | A307D |
| c.920C>G | c.C920G | A307G |
| c.920C>T | c.C920T | A307V |
| c.922A>C | c.A922C | K308Q |
| c.922A>G | c.A922G | K308E |
| c.923A>G | c.A923G | K308R |
| c.923A>T | c.A923T | K308I |
| c.924A>T or c.924A>C | c.A924T or c.A924C | K308N |
| c.925G>A | c.G925A | A309T |
| c.925G>C | c.G925C | A309P |
| c.926C>A | c.C926A | A309D |
| c.926C>T | c.C926T | A309V |
| c.928C>A | c.C928A | L310I |
| c.928C>G | c.C928G | L310V |
| c.928C>T | c.C928T | L310F |
| c.931C>A | c.C931A | L311I |
| c.931C>G | c.C931G | L311V |
| c.934C>A | c.C934A | Q312K |
| c.934C>G | c.C934G | Q312E |
| c.935A>G | c.A935G | Q312R |
| c.935A>T | c.A935T | Q312L |
| c.936G>T or c.936G>C | c.G936T or c.G936C | Q312H |
| c.937G>T | c.G937T | D313Y |
| c.[937G>T; 1232G>A] | c.G937T/G1232A | D313Y/G411D |
| c.938A>G | c.A938G | D313G |
| c.938A>T | c.A938T | D313V |
| c.939T>A | c.T939A | D313E |
| c.940A>G | c.A940G | K314E |
| c.941A>C | c.A941C | K314T |
| c.941A>T | c.A941T | K314M |
| c.942G>C | c.G942C | K314N |
| c.943G>A | c.G943A | D315N |
| c.943G>C | c.G943C | D315H |
| c.943G>T | c.G943T | D315Y |
| c.944A>C | c.A944C | D315A |
| c.944A>G | c.A944G | D315G |
| c.944A>T | c.A944T | D315V |
| c.946G>A | c.G946A | V316I |
| c.946G>C | c.G946C | V316L |
| c.947T>C | c.T947C | V316A |
| c.947T>G | c.T947G | V316G |
| c.949A>C | c.A949C | I317L |
| c.949A>G | c.A949G | I317V |
| c.950T>C | c.T950C | I317T |
| c.951T>G | c.T951G | I317M |
| c.952G>A | c.G952A | A318T |
| c.952G>C | c.G952C | A318P |
| c.953C>A | c.C953A | A318D |
| c.953C>T | c.C953T | A318V |
| c.955A>T | c.A955T | I319F |
| c.956T>C | c.T956C | I319T |
| c.957C>G | c.C957G | I319M |
| c.958A>C | c.A958C | N320H |
| c.959A>C | c.A959C | N320T |
| c.959A>G | c.A959G | N320S |
| c.959A>T | c.A959T | N320I |
| c.961C>A | c.C961A | Q321K |
| c.962A>G | c.A962G | Q321R |
| c.962A>T | c.A962T | Q321L |
| c.963G>C or c.963G>T | c.G963C or c.G963T | Q321H |
| c.964G>A | c.G964A | D322N |
| c.964G>C | c.G964C | D322H |
| c.965A>C | c.A965C | D322A |
| c.965A>T | c.A965T | D322V |
| c.966C>A or c.966C>G | c.C966A or c.C966G | D322E |
| c.967C>A | c.C967A | P323T |
| c.968C>G | c.C968G | P323R |
| c.970T>G | c.T970G | L324V |
| c.971T>G | c.T971G | L324W |
| c.973G>A | c.G973A | G325S |
| c.973G>C | c.G973C | G325R |
| c.973G>T | c.G973T | G325C |
| c.974G>C | c.G974C | G325A |
| c.974G>T | c.G974T | G325V |
| c.976A>C | c.A976C | K326Q |
| c.976A>G | c.A976G | K326E |
| c.977A>C | c.A977C | K326T |
| c.977A>G | c.A977G | K326R |
| c.977A>T | c.A977T | K326M |
| c.978G>C or c.978G>T | c.G978C or c.G978T | K326N |
| c.979C>G | c.C979G | Q327E |
| c.980A>C | c.A980C | Q327P |
| c.980A>T | c.A980T | Q327L |
| c.981A>T | c.A981T | Q327H |
| c.983G>C | c.G983C | G328A |
| c.985T>A | c.T985A | Y329N |
| c.985T>C | c.T985C | Y329H |
| c.985T>G | c.T985G | Y329D |
| c.986A>G | c.A986G | Y329C |
| c.986A>T | c.A986T | Y329F |
| c.988C>A | c.C988A | Q330K |
| c.988C>G | c.C988G | Q330E |
| c.989A>C | c.A989C | Q330P |
| c.989A>G | c.A989G | Q330R |
| c.990G>C | c.G990C | Q330H |
| c.991C>G | c.C991G | L331V |
| c.992T>A | c.T992A | L331H |
| c.992T>C | c.T992C | L331P |
| c.992T>G | c.T992G | L331R |
| c.994A>G | c.A994G | R332G |
| c.995G>C | c.G995C | R332T |
| c.995G>T | c.G995T | R332I |
| c.996A>T | c.A996T | R332S |
| c.997C>G | c.C997G | Q333E |
| c.998A>C | c.A998C | Q333P |
| c.998A>T | c.A998T | Q333L |
| c.1000G>C | c.G1000C | G334R |
| c.1001G>A | c.G1001A | G334E |
| c.1001G>T | c.G1001T | G334V |
| c.1003G>T | c.G1003T | D335Y |
| c.1004A>C | c.A1004C | D335A |
| c.1004A>G | c.A1004G | D335G |
| c.1004A>T | c.A1004T | D335V |
| c.1005C>G | c.C1005G | D335E |
| c.1006A>G | c.A1006G | N336D |
| c.1006A>T | c.A1006T | N336Y |
| c.1007A>C | c.A1007C | N336T |
| c.1007A>G | c.A1007G | N336S |
| c.1007A>T | c.A1007T | N336I |
| c.1009T>G | c.T1009G | F337V |
| c.1010T>A | c.T1010A | F337Y |
| c.1010T>C | c.T1010C | F337S |
| c.1010T>G | c.T1010G | F337C |
| c.1011T>A | c.T1011A | F337L |
| c.1012G>A | c.G1012A | E338K |
| c.1013A>C | c.A1013C | E338A |
| c.1013A>G | c.A1013G | E338G |
| c.1013A>T | c.A1013T | E338V |
| c.1014A>T | c.A1014T | E338D |
| c.1015G>A | c.G1015A | V339M |
| c.1016T>A | c.T1016A | V339E |
| c.1016T>C | c.T1016C | V339A |
| c.1021G>C | c.G1021C | E341Q |
| c.1022A>C | c.A1022C | E341A |
| c.1027C>A | c.C1027A | P343T |
| c.1027C>G | c.C1027G | P343A |
| c.1027C>T | c.C1027T | P343S |
| c.1028C>T | c.C 1028T | P343L |
| c.1030C>G | c.C1030G | L344V |
| c.1030C>T | c.C1030T | L344F |
| c.1031T>G | c.T1031G | L344R |
| c.1033T>C | c.T1033C | S345P |
| c.1036G>T | c.G1036T | G346C |
| c.1037G>A | c.G1037A | G346D |
| c.1037G>C | c.G1037C | G346A |
| c.1037G>T | c.G1037T | G346V |
| c.1039T>A | c.T1039A | L347I |
| c.1043C>A | c.C1043A | A348D |
| c.1046G>C | c.G1046C | W349S |
| c.1046G>T | c.G1046T | W349L |
| c.1047G>C | c.G1047C | W349C |
| c.1048G>A | c.G1048A | A350T |
| c.1048G>T | c.G1048T | A350S |
| c.1049C>G | c.C1049G | A350G |
| c.1049C>T | c.C1049T | A350V |
| c.1052T>A | c.T1052A | V351E |
| c.1052T>C | c.T1052C | V351A |
| c.1054G>A | c.G1054A | A352T |
| c.1054G>T | c.G1054T | A352S |
| c.1055C>G | c.C1055G | A352G |
| c.1055C>T | c.C1055T | A352V |
| c.1057A>T | c.A1057T | M353L |
| c.1058T>A | c.T1058A | M353K |
| c.1058T>C | c.T1058C | M353T |
| c.1061T>A | c.T1061A | I354K |
| c.1061T>G | c.T1061G | I354R |
| c.1063A>C | c.A1063C | N355H |
| c.1063A>G | c.A1063G | N355D |
| c.1063A>T | c.A1063T | N355Y |
| c.1064A>G | c.A1064G | N355S |
| c.1066C>G | c.C1066G | R356G |
| c.1066C>T | c.C 1066T | R356W |
| c.1067G>A | c.G1067A | R356Q |
| c.1067G>C | c.G1067C | R356P |
| c.1067G>T | c.G1067T | R356L |
| c.1069C>G | c.C1069G | Q357E |
| c.1072G>C | c.G1072C | E358Q |
| c.1073A>C | c.A1073C | E358A |
| c.1073A>G | c.A1073G | E358G |
| c.1074G>T or c.1074G>C | c.G1074T or c.G1074C | E358D |
| c.1075A>C | c.A1075C | I359L |
| c.1075A>G | c.A1075G | I359V |
| c.1075A>T | c.A1075T | I359F |
| c.1076T>A | c.T1076A | I359N |
| c.1076T>C | c.T1076C | I359T |
| c.1076T>G | c.T1076G | I359S |
| c.1078G>A | c.G1078A | G360S |
| c.1078G>C | c.G1078C | G360R |
| c.1078G>T | c.G1078T | G360C |
| c.1079G>A | c.G1079A | G360D |
| c.1079G>C | c.G1079C | G360A |
| c.1082G>A | c.G1082A | G361E |
| c.1082G>C | c.G1082C | G361A |
| c.1084C>A | c.C1084A | P362T |
| c.1084C>G | c.C1084G | P362A |
| c.1084C>T | c.C1084T | P362S |
| c.1085C>A | c.C1085A | P362H |
| c.1085C>G | c.C1085G | P362R |
| c.1085C>T | c.C1085T | P362L |
| c.1087C>A | c.C1087A | R363S |
| c.1087C>G | c.C1087G | R363G |
| c.1087C>T | c.C1087T | R363C |
| c.1088G>A | c.G1088A | R363H |
| c.1088G>T | c.G1088T | R363L |
| c.1090T>C | c.T1090C | S364P |
| c.1091C>G | c.C1091G | S364C |
| c.1093T>A | c.T1093A | Y365N |
| c.1093T>G | c.T1093G | Y365D |
| c.1094A>C | c.A1094C | Y365S |
| c.1094A>T | c.A1094T | Y365F |
| c.1096A>C | c.A1096C | T366P |
| c.1096A>T | c.A1096T | T366S |
| c.1097C>A | c.C1097A | T366N |
| c.1097C>T | c.C1097T | T366I |
| c.1099A>C | c.A1099C | I367L |
| c.1099A>T | c.A1099T | I367F |
| c.1101C>G | c.C1101G | I367M |
| c.1102G>A | c.G1102A | A368T |
| c.1102G>C | c.G1102C | A368P |
| c.1103C>G | c.C1103G | A368G |
| c.1105G>A | c.G1105A | V369I |
| c.1105G>C | c.G1105C | V369L |
| c.1105G>T | c.G1105T | V369F |
| c.1106T>C | c.T1106C | V369A |
| c.1106T>G | c.T1106G | V369G |
| c.1108G>A | c.G1108A | A370T |
| c.1108G>C | c.G1108C | A370P |
| c.1109C>A | c.C1109A | A370D |
| c.1109C>G | c.C1109G | A370G |
| c.1109C>T | c.C1109T | A370V |
| c.1111T>A | c.T1111A | S371T |
| c.1112C>G | c.C1112G | S371C |
| c.1117G>A | c.G1117A | G373S |
| c.1117G>T | c.G1117T | G373C |
| c.1118G>C | c.G1118C | G373A |
| c.1120A>G | c.A1120G | K374E |
| c.1121A>C | c.A1121C | K374T |
| c.1121A>G | c.A1121G | K374R |
| c.1121A>T | c.A1121T | K374I |
| c.1123G>C | c.G1123C | G375R |
| c.1124G>A | c.G1124A | G375E |
| c.1124G>C | c.G1124C | G375A |
| c.1126G>A | c.G1126A | V376M |
| c.1126G>C | c.G1126C | V376L |
| c.1127T>A | c.T1127A | V376E |
| c.1127T>G | c.T1127G | V376G |
| c.1129G>A | c.G1129A | A377T |
| c.1129G>C | c.G1129C | A377P |
| c.1129G>T | c.G1129T | A377S |
| c.1130C>G | c.C1130G | A377G |
| c.1135A>G | c.A1135G | N379D |
| c.1136A>C | c.A1136C | N379T |
| c.1136A>T | c.A1136T | N379I |
| c.1137T>A | c.T1137A | N379K |
| c.1138C>A | c.C1138A | P380T |
| c.1138C>G | c.C1138G | P380A |
| c.1139C>A | c.C1139A | P380H |
| c.1139C>G | c.C1139G | P380R |
| c.1139C>T | c.C1139T | P380L |
| c.1142C>A | c.C1142A | A381D |
| c.1147T>A | c.T1147A | F383I |
| c.1148T>A | c.T1148A | F383Y |
| c.1148T>G | c.T1148G | F383C |
| c.1150A>T | c.A1150T | I384F |
| c.1151T>C | c.T1151C | I384T |
| c.1152C>G | c.C1152G | I384M |
| c.1153A>G | c.A1153G | T385A |
| c.1154C>T | c.C1154T | T385I |
| c.1156C>A | c.C1156A | Q386K |
| c.1157A>T | c.A1157T | Q386L |
| c.1158G>C | c.G1158C | Q386H |
| c.1159C>A | c.C1159A | L387I |
| c.1159C>T | c.C1159T | L387F |
| c.1160T>A | c.T1160A | L387H |
| c.1160T>G | c.T1160G | L387R |
| c.1162C>A | c.C1162A | L388I |
| c.1162C>G | c.C1162G | L388V |
| c.1162C>T | c.C1162T | L388F |
| c.1163T>A | c.T1163A | L388H |
| c.1163T>G | c.T1163G | L388R |
| c.1168G>A | c.G1168A | V390M |
| c.1171A>C | c.A1171C | K391Q |
| c.1171A>G | c.A1171G | K391E |
| c.1172A>C | c.A1172C | K391T |
| c.1172A>G | c.A1172G | K391R |
| c.1172A>T | c.A1172T | K391I |
| c.1173A>T | c.A1173T | K391N |
| c.1174A>G | c.A1174G | R392G |
| c.1174A>T | c.A1174T | R392W |
| c.1175G>A | c.G1175A | R392K |
| c.1175G>C | c.G1175C | R392T |
| c.1175G>T | c.G1175T | R392M |
| c.1177A>C | c.A1177C | K393Q |
| c.1177A>G | c.A1177G | K393E |
| c.1178A>C | c.A1178C | K393T |
| c.1179G>C | c.G1179C | K393N |
| c.1180C>A | c.C1180A | L394I |
| c.1181T>A | c.T1181A | L394Q |
| c.1181T>C | c.T1181C | L394P |
| c.1181T>G | c.T1181G | L394R |
| c.1183G>C | c.G1183C | G395R |
| c.1184G>A | c.G1184A | G395E |
| c.1184G>C | c.G1184C | G395A |
| c.1186T>A | c.T1186A | F396I |
| c.1186T>G | c.T1186G | F396V |
| c.1187T>G | c.T1187G | F396C |
| c.1188C>G | c.C1188G | F396L |
| c.1189T>A | c.T1189A | Y397N |
| c.1189T>C | c.T1189C | Y397H |
| c.1190A>C | c.A1190C | Y397S |
| c.1190A>G | c.A1190G | Y397C |
| c.1190A>T | c.A1190T | Y397F |
| c.1192G>A | c.G1192A | E398K |
| c.1192G>C | c.G1192C | E398Q |
| c.1193A>G | c.A1193G | E398G |
| c.1195T>A | c.T1195A | W399R |
| c.1195T>G | c.T1195G | W399G |
| c.1198A>C | c.A1198C | T400P |
| c.1198A>G | c.A1198G | T400A |
| c.1198A>T | c.A1198T | T400S |
| c.1199C>A | c.C1199A | T400N |
| c.1199C>T | c.C1199T | T400I |
| c.1201T>A | c.T1201A | S401T |
| c.1201T>G | c.T1201G | S401A |
| c.1202_1203insGACTTC | c.1202_1203insGACTTC | p.T400_S401dup |
| c.1202C>T | c.C1202T | S401L |
| c.1204A>G | c.A1204G | R402G |
| c.1204A>T | c.A1204T | R402W |
| c.1205G>C | c.G1205C | R402T |
| c.1205G>T | c.G1205T | R402M |
| c.1206G>C | c.G1206C | R402S |
| c.1207T>G | c.T1207G | L403V |
| c.1208T>C | c.T1208C | L403S |
| c.1209A>T | c.A1209T | L403F |
| c.1210A>G | c.A1210G | R404G |
| c.1211G>A | c.G1211A | R404K |
| c.1211G>C | c.G1211C | R404T |
| c.1211G>T | c.G1211T | R404I |
| c.1212A>T | c.A1212T | R404S |
| c.1213A>G | c.A1213G | S405G |
| c.1216C>G | c.C1216G | H406D |
| c.1217A>T | c.A1217T | H406L |
| c.1218C>G | c.C1218G | H406Q |
| c.1219A>T | c.A1219T | I407L |
| c.1220T>C | c.T1220C | I407T |
| c.1221A>G | c.A1221G | I407M |
| c.1222A>C | c.A1222C | N408H |
| c.1222A>G | c.A1222G | N408D |
| c.1222A>T | c.A1222T | N408Y |
| c.1223A>C | c.A1223C | N408T |
| c.1225C>A | c.C1225A | P409T |
| c.1225C>G | c.C1225G | P409A |
| c.1225C>T | c.C1225T | P409S |
| c.1226C>T | c.C1226T | P409L |
| c.1228A>G | c.A1228G | T410A |
| c.1228A>T | c.A1228T | T410S |
| c.1229C>T | c.C1229T | T410I |
| c.1231G>A | c.G1231A | G411S |
| c.1231G>T | c.G1231T | G411C |
| c.1232G>A | c.G1232A | G411D |
| c.1232G>C | c.G1232C | G411A |
| c.1232G>T | c.G1232T | G411V |
| c.1234A>C | c.A1234C | T412P |
| c.1234A>G | c.A1234G | T412A |
| c.1234A>T | c.A1234T | T412S |
| c.1235C>A | c.C1235A | T412N |
| c.1235C>T | c.C1235T | T412I |
| c.1237G>A | c.G1237A | V413I |
| c.1237G>T | c.G1237T | V413F |
| c.1238T>G | c.T1238G | V413G |
| c.1240T>G | c.T1240G | L414V |
| c.1242G>C | c.G1242C | L414F |
| c.1243C>A | c.C1243A | L415I |
| c.1244T>A | c.T1244A | L415H |
| c.1246C>G | c.C1246G | Q416E |
| c.1247A>T | c.A1247T | Q416L |
| c.1248G>C | c.G1248C | Q416H |
| c.1249C>A | c.C1249A | L417I |
| c.1252G>A | c.G1252A | E418K |
| c.1252G>C | c.G1252C | E418Q |
| c.1253A>C | c.A1253C | E418A |
| c.1253A>G | c.A1253G | E418G |
| c.1254A>T | c.A1254T | E418D |
| c.1255A>G | c.A1255G | N419D |
| c.1255A>T | c.A1255T | N419Y |
| c.1256A>C | c.A1256C | N419T |
| c.1256A>G | c.A1256G | N419S |
| c.1256A>T | c.A1256T | N419I |
| c.1258A>C | c.A1258C | T420P |
| c.1258A>T | c.A1258T | T420S |
| c.1259C>A | c.C1259A | T420K |
| c.1259C>G | c.C1259G | T420R |
| c.1261A>G | c.A1261G | M421V |
| c.1261A>T | c.A1261T | M421L |
| c.1262T>A | c.T1262A | M421K |
| c.1262T>C | c.T1262C | M421T |
| c.1262T>G | c.T1262G | M421R |
| c.1263G>C | c.G1263C | M421I |
| c.1265A>C | c.A1265C | Q422P |
| c.1267A>T | c.A1267T | M423L |
| c.1268T>A | c.T1268A | M423K |
| c.1268T>C | c.T1268C | M423T |
| c.1269G>C | c.G1269C | M423I |
| c.1271C>T | c.C1271T | S424L |
| c.1275A>C | c.A1275C | L425F |
| c.1279G>A | c.G1279A | D427N |
| c.1286T>G | c.T1286G | L429R |

### Dosing, Formulation and Administration

The Fabry patient is administered migalastat or salt thereof at a frequency of once every other day (also referred to as "QOD"). In various embodiments, the doses described herein pertain to migalastat hydrochloride or an equivalent dose of migalastat or a salt thereof other than the hydrochloride salt. In some embodiments, these doses pertain to the free base of migalastat. In alternate embodiments, these doses pertain to a salt of migalastat. In further embodiments, the salt of migalastat is migalastat hydrochloride. The administration of migalastat or a salt of migalastat is referred to herein as "migalastat therapy".

The effective amount of migalastat or salt thereof can be in the range from about 100 mg FBE to about 150 mg FBE. Exemplary doses include about 100 mg FBE, about 105 mg FBE, about 110 mg FBE, about 115 mg FBE, about 120 mg FBE, about 123 mg FBE, about 125 mg FBE, about 130 mg FBE, about 135 mg FBE, about 140 mg FBE, about 145 mg FBE or about 150 mg FBE.

Again, it is noted that 150 mg of migalastat hydrochloride is equivalent to 123 mg of the free base form of migalastat. Thus, in one or more embodiments, the dose is 150 mg of migalastat hydrochloride or an equivalent dose of migalastat or a salt thereof other than the hydrochloride salt, administered at a frequency of once every other day. As set forth above, this dose is referred to as 123 mg FBE of migalastat. In further embodiments, the dose is 150 mg of migalastat hydrochloride administered at a frequency of once every other day. In other embodiments, the dose is 123 mg of the migalastat free base administered at a frequency of once every other day.

In various embodiments, the effective amount is about 122 mg, about 128 mg, about 134 mg, about 140 mg, about 146 mg, about 150 mg, about 152 mg, about 159 mg, about 165 mg, about 171 mg, about 177 mg or about 183 mg of migalastat hydrochloride.

Accordingly, in various embodiments, migalastat therapy includes administering 123 mg FBE at a frequency of once every other day, such as 150 mg of migalastat hydrochloride every other day. is for at least 18 months as defined in the appended claims.

The administration of migalastat or salt thereof In one or more embodiments, the migalastat or salt thereof is administered for a duration of at least 20, 24, 30 or 36 months or at least 2, 3, 4 or 5 years. In various embodiments, the migalastat therapy is a long-term migalastat therapy of at least about 2, 3, 4 or 5 years.

Administration of migalastat or salt thereof according to the present invention may be in a formulation suitable for any route of administration, but is preferably administered in an oral dosage form such as a tablet, capsule or solution. As one example, the patient is orally administered capsules each containing 150 mg migalastat hydrochloride or an equivalent dose of migalastat or a salt thereof other than the hydrochloride salt.

In some embodiments, the PC ( migalastat or salt thereof) is administered orally. In one or more embodiments, the PC ( migalastat or salt thereof) is administered by injection. The PC may be accompanied by a pharmaceutically acceptable carrier, which may depend on the method of administration.

In one or more embodiments, the PC ( migalastat or salt thereof) is administered as monotherapy, and can be in a form suitable for any route of administration, including *e.g*., orally in the form tablets or capsules or liquid, or in sterile aqueous solution for injection. In other embodiments, the PC is provided in a dry lyophilized powder to be added to the formulation of the replacement enzyme during or immediately after reconstitution to prevent enzyme aggregation *in vitro* prior to administration.

When the PC ( migalastat or salt thereof) is formulated for oral administration, the tablets or capsules can be prepared by conventional means with pharmaceutically acceptable excipients such as binding agents (*e.g*., pregelatinized maize starch, polyvinylpyrrolidone or hydroxypropyl methylcellulose); fillers (*e.g.,* lactose, microcrystalline cellulose or calcium hydrogen phosphate); lubricants (*e.g*., magnesium stearate, talc or silica); disintegrants (*e.g*., potato starch or sodium starch glycolate); or wetting agents (*e.g*., sodium lauryl sulfate). The tablets may be coated by methods well known in the art. Liquid preparations for oral administration may take the form of, for example, solutions, syrups or suspensions, or they may be presented as a dry product for constitution with water or another suitable vehicle before use. Such liquid preparations may be prepared by conventional means with pharmaceutically acceptable additives such as suspending agents (*e.g*., sorbitol syrup, cellulose derivatives or hydrogenated edible fats); emulsifying agents (*e.g*., lecithin or acacia); non-aqueous vehicles (*e.g*., almond oil, oily esters, ethyl alcohol or fractionated vegetable oils); and preservatives (*e.g*., methyl or propyl-p- hydroxybenzoates or sorbic acid). The preparations may also contain buffer salts, flavoring, coloring and sweetening agents as appropriate. Preparations for oral administration may be suitably formulated to give controlled release of the active chaperone compound.

The pharmaceutical formulations of the PC ( migalastat or salt thereof) suitable for parenteral/injectable use generally include sterile aqueous solutions (where water soluble), or dispersions and sterile powders for the extemporaneous preparation of sterile injectable solutions or dispersion. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. Prevention of the action of microorganisms can be brought about by various antibacterial and antifungal agents, for example, parabens, chlorobutanol, phenol, benzyl alcohol, sorbic acid, and the like. In many cases, it will be reasonable to include isotonic agents, for example, sugars or sodium chloride. Prolonged absorption of the injectable compositions can be brought about by the use in the compositions of agents delaying absorption, for example, aluminum monosterate and gelatin.

Sterile injectable solutions are prepared by incorporating the purified enzyme (if any) and the PC ( migalastat or salt thereof) in the required amount in the appropriate solvent with various of the other ingredients enumerated above, as required, followed by filter or terminal sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

The formulation can contain an excipient. Pharmaceutically acceptable excipients which may be included in the formulation are buffers such as citrate buffer, phosphate buffer, acetate buffer, bicarbonate buffer, amino acids, urea, alcohols, ascorbic acid, and phospholipids; proteins, such as serum albumin, collagen, and gelatin; salts such as EDTA or EGTA, and sodium chloride; liposomes; polyvinylpyrollidone; sugars, such as dextran, mannitol, sorbitol, and glycerol; propylene glycol and polyethylene glycol *(e.g.,* PEG-4000, PEG-6000); glycerol; glycine or other amino acids; and lipids. Buffer systems for use with the formulations include citrate; acetate; bicarbonate; and phosphate buffers. Phosphate buffer is a preferred embodiment.

The route of administration of the chaperone compound may be oral or parenteral, including intravenous, subcutaneous, intra-arterial, intraperitoneal, ophthalmic, intramuscular, buccal, rectal, vaginal, intraorbital, intracerebral, intradermal, intracranial, intraspinal, intraventricular, intrathecal, intracisternal, intracapsular, intrapulmonary, intranasal, transmucosal, transdermal, or via inhalation.

Administration of the above-described parenteral formulations of the chaperone compound may be by periodic injections of a bolus of the preparation, or may be administered by intravenous or intraperitoneal administration from a reservoir which is external *(e.g.,* an i.v. bag) or internal *(e.g.,* a bioerodable implant).

Embodiments relating to pharmaceutical formulations and administration may be combined with any of the other embodiments of the invention, for example embodiments relating to methods of treating patients with Fabry disease, methods of treating ERT-naïve Fabry patients, methods of treating ERT-experienced Fabry patients, methods of reducing the risk of CBV events, methods of reducing the risk of composite clinical outcomes, methods of assessing symptoms or outcomes of a patient or groups of patients, methods of evaluating a treatment therapy, methods of enhancing α-Gal A in a patient diagnosed with or suspected of having Fabry disease, use of a pharmacological chaperone for α-Gal A for the manufacture of a medicament for treating a patient diagnosed with Fabry disease or to a pharmacological chaperone for α-Gal A for use in treating a patient diagnosed with Fabry disease as well as embodiments relating to amenable mutations, the PCs and suitable dosages thereof.

In one or more embodiments, the PC ( migalastat or salt thereof) is administered in combination with ERT. ERT increases the amount of protein by exogenously introducing wild-type or biologically functional enzyme by way of infusion. This therapy has been developed for many genetic disorders, including LSDs such as Fabry disease, as referenced above. After the infusion, the exogenous enzyme is expected to be taken up by tissues through non-specific or receptor-specific mechanism. In general, the uptake efficiency is not high, and the circulation time of the exogenous protein is short. In addition, the exogenous protein is unstable and subject to rapid intracellular degradation as well as having the potential for adverse immunological reactions with subsequent treatments. In one or more embodiments, the chaperone is administered at the same time as replacement enzyme (*e.g.,* replacement α-Gal A). In some embodiments, the chaperone is co-formulated with the replacement enzyme (*e.g*., replacement α-Gal A).

In one or more embodiments, a patient is switched from ERT to migalastat therapy. In some embodiments, a patient on ERT is identified, the patient's ERT is discontinued, and the patient begins receiving migalastat therapy. The migalastat therapy can be in accordance with any of the methods described herein.

### Composite Clinical Outcomes

The dosing regimens described herein can reduce the risk of composite clinical outcomes (CCOs) in Fabry patients. As described in further detail in the Examples below, Phase 3 studies have found that migalastat therapy reduces the incidence of CCOs in Fabry patients. Accordingly, migalastat therapy can be used to reduce the risk of CCOs in Fabry patients and/or treat Fabry patients that have a high risk of CCOs, including for patients that have a history of cardiovascular, renal or cerebrovascular events or patients that do not have a history of these events.

The CCO comprises renal events, cardiac events, cerebrovascular events and death. In one or more embodiments, the renal events comprise one or more of: a decrease in eGFR_{CKD-EPI} ≥ 15 mL/min/1.73 m², with the decreased eGFR <90 mL/min/1.73 m² relative to baseline; or an increase in 24-hour urine protein ≥33%, with elevated protein ≥300 mg relative to baseline. In one or more embodiments, the cardiac events comprise one or more of: myocardial infarction; unstable cardiac angina; new symptomatic arrhythmia requiring antiarrhythmic medication, direct current cardioversion, pacemaker, or defibrillator implantation; or congestive heart failure [New York Association Class III or IV]. In one or more embodiments, the cerebrovascular events comprise one or more of stroke or transient ischemic attack.

In one or more embodiments, the incidence rate of CCOs is less than 1.0 per patient•year, such as less than 0.9, 0.8, 0.7, 0.6, 0.5 or 0.4 per patient•year.

In one or more embodiments, the migalastat therapy provides a lower CCO incidence rate than a different treatment therapy. In one or more embodiments, the other treatment therapy comprises one or more of ERT, substrate reduction therapy or gene therapy. In one or more embodiments, the other therapy is ERT.

In one or more embodiments, the CCO incidence rate is evaluated after 18 months of treatment. In one or more embodiments, the CCO incidence rate is evaluated over long-term treatment periods of at least 2, 3, 4 or more years.

In one or more embodiments, the median time to first CCO is greater than 0.5 years, such as greater than 6 months, 7 months, 8 months, 9 months, 10 months, 11 months, 12 months or 1 year.

In one or more embodiments, the migalastat therapy provides a longer time to first CCO than a different treatment therapy. In one or more embodiments, the other treatment therapy comprises one or more of ERT, substrate reduction therapy or gene therapy. In one or more embodiments, the other therapy is ERT.

### EXAMPLES

### EXAMPLE 1: Composite Clinical Outcomes During Migalastat Therapy

This example describes the incidence of composite clinical outcomes (CCOs) in migalastat-treated patients who were previously treated with ERT.

### Study Designs

The analysis included data from 3 Phase 3 clinical trials with the data cutoff of May 25, 2019 as shown below. The analysis included ERT-experienced patients with migalastat-amenable GLA variants who had received migalastat in ATTRACT and the subsequent open-label extension (OLE) studies (AT1001-041 [NCT01458119] and/or AT1001-042, [NCT02194985]). Patients initiated ERT ≥12 months prior to the study.

ATTRACT (AT1001-012, NCT01218659) was a phase 3, open-label, active-controlled study to compare the efficacy and safety of 18 months of migalastat HCl 150 mg QOD versus ERT, followed by a 12-month OLE of migalastat, in ERT-treated patients with migalastat-amenable GLA variants.

AT1001-041 (NCT01458119) was a long-term OLE study evaluating the long-term safety and efficacy of migalastat in patients completing a previous migalastat study.

AT1001-042 (NCT02194985) is an ongoing, long-term OLE study evaluating the long-term safety and efficacy of migalastat in patients who participated in AT1001-012 or AT1001-041.

Clinical Studies Included in the Analysis

Baseline

Screening and Randomization
68 screened
8 did not meet inclusion criteria
60 randomized 1.5:1
Stratified by sex and proteinuria^a

Open-Label

Migalastat 150 mg QOD
All treated patients, n=36
Amenable^b patients, n=34
2 discontinuations

ERT^C
Agalsidase alfa or agalsidase beta
3 consent withdrawn prior to 1st dose
All treated patients, n=21
Amenable^b patients, n=19
3 discontinuations

Month 18

Completed period:
All patients, n=34
Amenable^b patients, n=32
Declined extension, n=1

Completed period:
All patients, n=18
Amenable^b patients, n=16
Declined extension, n=3

Open-Label
Extension

Migalastat 150 mg QOD
All patients, n=33
Amenable^b patients, n=31
3 discontinuations

Migalastat 150 mg QOD
All patients, n=15
Amenable^b patients, n=15
3 discontinuations

Month 30

Completed study:
All patients, n=30
Amenable^b patients, n=30

Completed study:
All patients, n=12
Amenable^b patients, n=12

Up to 5 years

Option to Enroll
in a Separate
Open-Label Extension

^aProteinuria stratification: high, ≥0.1 g/24 h; low, <0.1 g/24 h.

^bDetermination of amenability was based on the Good Laboratory Practice–validated
migalastat amenability assay, which became available during the study. Therefore, some
enrolled patients were later determined to be not amenable.
^cEight (38%) and 13 (62%) patients were receiving agalsidase beta and agalsidase alfa,
respectively.

CCOs were defined prior to the planned analysis and included:
- Renal events (a decrease in eGFR_{CKD-EPI} ≥15 mL/min/1.73 m², with the decreased eGFR <90 mL/min/1.73 m² relative to baseline; or an increase in 24-hour urine protein ≥33%, with elevated protein ≥300 mg relative to baseline)
- Cardiac events (myocardial infarction; unstable cardiac angina; new symptomatic arrhythmia requiring antiarrhythmic medication, direct current cardioversion, pacemaker, or defibrillator implantation; congestive heart failure [New York Association Class III or IV])
- Cerebrovascular events (stroke or transient ischemic attack)
- Death

### Data Analysis

Exposure to migalastat was calculated from the day of the first dose to the last available data point or the date of discontinuation; for this analysis, the exposure to ERT comprised the duration of participation in the ATTRACT study, and lasted up to 18 months.

Incidence rates were calculated for renal, cerebrovascular, and cardiac events separately and for the composite clinical outcomes. All individual events were accounted for when calculating incidence. Median times to the first clinical event were calculated based on the same data used for the incidence rates.

A direct comparison between migalastat and ERT was based on the 18 months exposure (the ERT data were from first 18 months in ATTRACT only); in addition, the incidence of CCOs for the whole follow-up period was calculated for migalastat (ATTRACT and subsequent OLE studies)

The long-term incidence for migalastat was calculated based on all available data for migalastat.

### Results

### Demographics and Baseline Characteristics

The mean (standard deviation) age of patients was 49.4 (14.1) years; on average, patients received 3.4 years of ERT prior to migalastat treatment (Table 2). The median (Q1-Q3) migalastat exposure was 4.8 (2.1, 5.5) years; individual patient values ranged from 0.1 to 7.2 years. Patients received migalastat at initiation of ATTRACT or switched from ERT after 18 months.

**Table 2. Patient Demographics and Baseline Characteristics^{a}**

| **Parameters** | | **ERT-experienced Patients N=49** | | |
|---|---|---|---|---|
| | | **Overall (N=49)** | **Males n=19** | **Females n=30** |
| **Age, years** | | | | |
| | Mean (SD) | 49.4 (14.1) | 48.1 (14.7) | 50.2 (14.0) |
| | Median (range) | 53.0 (18.0-70.0) | 48.0 (18.0-67.0) | 53.5 (18.0-70.0) |

| **Years since Fabry diagnosis, years** | | | | |
|---|---|---|---|---|
| | Mean (SD) | 11.6 (12.0) | 9.2 (10.6) | 13.0 (12.8) |
| | Median (range) | 5.7 (1.2-42.9) | 4.4 (1.9-38.2) | 6.3 (1.2-42.9) |

| **Duration of ERT exposure, years** | | | | |
|---|---|---|---|---|
| | Mean (SD) | 3.4 (2.4) | 3.5 (2.3) | 3.4 (2.5) |
| | Median (range) | 2.3 (0.3-8.6) | 2.4 (0.5-8.5) | 2.2 (0.3-8.6) |
| **eGFR_{CKD-EPI}, mean (SD), mL/min/1.73 m²** | | 89.3 (20.5) | 90.1 (21.6) | 88.8 (20.1) |
| **LVMi, mean (SD), g/m²** | | 93.0 (25.6) | 107.5 (29.5) | 84.5 (18.9) |

| | | | | |
|---|---|---|---|---|
| ^{a}Values were from the beginning of migalastat treatment. | | | | |

### Incidence of Clinical Events

During the first 18 months of migalastat or ERT treatment, the incidence rate of CCOs was 0.41 per patient•year for migalastat and 0.85 per patient•year for ERT. In male and female patients receiving migalastat treatment, the incidence rate of CCOs during the first 18 months was 0.50 and 0.34 per patient•year, respectively (Table 3).

The incidence rate of CCOs remained low during long-term follow-up and was 0.45 per patient•year at the data cutoff. During long-term follow-up, the incidence rate of CCOs was 0.56 and 0.37 per patient•year at the data cutoff in male and female patients, respectively (Table 3).

**Table 3. Incidence Rate of Composite Clinical Outcomes by Treatment and Sex**

| Per patient•year | **ERT At 18 months** | | **Migalastat At 18 months** | | **Migalastat At 4.8 years^{a}** | |
|---|---|---|---|---|---|---|
| | **Males N=8** | **Females N=10** | **Males N=19** | **Females N=30** | **Males N=19** | **Females N=30** |
| **Composite clinical outcome** | 0.68 | 0.98 | 0.50 | 0.34 | 0.56 | 0.37 |
| **Renal event** | 0.58 | 0.56 | 0.43 | 0.29 | 0.51 | 0.33 |
| **Cardiac event** | 0 | 0.42 | 0.07 | 0.05 | 0.04 | 0.02 |
| **Cerebrovascular event** | 0.10 | 0 | 0 | 0 | 0.01 | 0.02 |
| **Death** | 0 | 0 | 0 | 0 | 0 | 0 |

### Time to Clinical Events

Overall, median time to first CCO was 1.17 and 0.25 years with migalastat and ERT, respectively. Median time to first CCOs by treatment and sex are shown in Table 4.

**Table 4. Median Time to First Composite Clinical Outcome by Treatment and Sex**

| Years | **ERT (0-18 months)** | | **Migalastat (0-4.8 years)** | |
|---|---|---|---|---|
| | **Males** | **Females** | **Males** | **Females** |
| **Composite clinical outcome** | 0.25 | 0.35 | 1.30 | 1.15 |

As can be seen from the tables above, the incidence rate of CCOs in patients on migalastat was lower and time to first CCO was longer with migalastat compared with ERT during the 18-month treatment period in the ATTRACT study. This CCO incidence rate was maintained for patients who continued migalastat for up to 7.2 years (median follow-up: 4.8 years) in the open label extension. These data demonstrated long-term benefits of migalastat treatment in ERT-experienced patients with Fabry disease and amenable GLA mutations.

### EXAMPLE 2: FABry Disease Patient-Reported Outcome-Gastrointestinal (FABPRO-GI)

This example describes the development of a new Fabry disease-specific gastrointestinal (GI) outcomes instrument.

### Current GI Instruments

The Gastrointestinal Symptom Rating Scale has been used to assess GI symptoms in a clinical trial of migalastat; however, no validated Fabry disease-specific instruments to fully evaluate the impact of treatments on Fabry disease-related GI symptoms are available (Table 5).

**Table 5. Instruments Used to Assess GI Symptoms in Patients With Fabry Disease**

| **Instrument** | **Advantages** | **Disadvantages** |
|---|---|---|
| Gastrointestinal Symptom Rating Scale | • Assesses a broad range of GI symptoms | • Does not assess multiple items for diarrhea (e.g., frequency and severity) |
| | • Can be used in its entirety or as a pool of items from which appropriate items can be selected | |
| | | • Not validated for GI symptoms associated with Fabry disease |
| | • Is a patient-reported outcome | |
| Rome III criteria (for adult and pediatric patients) | • Can be reported by caregivers or patients | • Recommends that symptoms originate 6 months prior |
| | • Validated for several functional GI disorders | to diagnosis and be active for 3 months |
| | | • Not validated for GI symptoms associated with Fabry disease |

| | | |
|---|---|---|
| ^{a}Diagnostic scoring system for irritable bowel syndrome given that Fabry disease-related GI symptoms mimic irritable bowel syndrome | | |

Based on the shortcomings of these other instruments, the FABry disease Patient-Reported Outcome-Gastrointestinal (FABPRO-GI) instrument was developed to be a literature review- and quantitative analysis-based instrument designed to assess GI symptoms in patients with Fabry disease.

### Methods

The FABPRO-GI instrument was drafted based on results from a comprehensive literature review, expert advice meetings, and patient concept elicitation interviews consistent with measurement best practices and regulatory guidance.

Patient perspectives regarding Fabry-disease related GI symptoms were obtained via concept elicitation interviews. 15 patients who had self-reported Fabry disease, were fluent in English, and aged ≥16 years with ≥1 self-reported GI symptom in the 14 days prior to study entry were included in the study After development of the initial FABPRO-GI instrument, cognitive debriefing interviews with an additional 15 patients were conducted to evaluate its content and produce the final FABPRO-GI instrument. Demographics of patients interviewed in concept elicitation interviews and cognitive debriefing interviews are presented in Table 6.

**Table 6. Demographics and Disease Characteristics of Patients With Fabry Disease Participating in CEIs and CDIs**

| | | **CEIs (N=17)** | **CDIs (N=15)** |
|---|---|---|---|
| Age, years, mean (SD) | | 33.7 (14.2) | 39.6 (16.7) |
| Female, n (%) | | 10 (58.8) | 11 (73.3) |
| Race, n (%) | | | |
| | White | 11 (64.7) | 13 (86.7) |
| | Black or African American | - | 2 (13.3) |
| | Other | 5 (29.4) | - |
| | Not answered | 1 (5.8) | - |
| Time since diagnosis, mean (SD) | | 11.8 (12.2) | 7.9 (6.2) |
| Fabry disease-related GI symptom severity, n (%)^{a} | | | |
| | Very mild | 1 (5.9) | 1 (6.7) |
| | Mild | 3 (17.6) | 2 (13.3) |
| | Moderate | 11 (64.7) | 11 (73.3) |
| | Severe | 1(5.9) | 1 (6.7) |
| | Very severe | 1 (5.9) | - |

| | | | |
|---|---|---|---|
| CDI=cognitive debriefing interviews; CEI=concept elicitation interviews; NSAIDs=nonsteroidal anti-inflammatory drugs; ^{a}Patient-reported; ^{b}Not mutually exclusive; ^{c}Enzyme replacement therapy was excluded; ^{d}For example, phenytoin, carbamazepine, or gabapentin; ^{e}For example, narcotics, opioids such as codeine, hydrocodone, Demerol, OxyContin, or Percocet. | | | |

Three sets of conceptual models were derived from the literature review, expert advice meetings, and patient concept elicitation interviews, which show largely overlapping GI symptoms:

Literature
Review
Expert Advice
Meetings
(N=5)
Chest pain
Indigestion
Abdominal pain
Weight loss
Early satiety
Acid reflux
Poor appetite
Abdominal cramps
Bloating
Constipation
Diarrhea
Gas
Nausea
Vomiting
Burping
Stomach pain
Heartburn
Upset stomach
Gas pain
Frequent bowel
movements
Patient Interviews
(N=17)

The initial FABPRO-GI instrument was revised based on feedback from cognitive debriefing interviews to produce the final 11-item FABPRO-GI instrument (Table 7).

**Table 7. Final Items of the Final FABPRO-GI Instrument^{a}**

| **Domain** | **Item** |
|---|---|
| Disease-related GI symptom severity | 1. Over the past 24 hours, how severe was your **worst bloating?** |
| | 2. Over the past 24 hours, how severe was your **worst stomach pain?** |
| | 3. Over the past 24 hours, how severe was your **worst cramping?** |
| | 4. Over the past 24 hours, how severe was your **worst nausea?** |
| | 5. Over the past 24 hours, how severe was your **worst acid reflux?** |
| | 6. Over the past 24 hours, how severe was your **worst heartburn?** |
| | 8. Over the past 24 hours, how severe was your **worst constipation?** |
| | 12. Over the past 24 hours, how severe was your **worst diarrhea?** |
| Frequency of bowel movements | 9. Over the past 24 hours, how many **bowel movements** did you have? |
| Frequency of diarrhea | 10. Over the past 24 hours, how many times did you have **diarrhea?** |
| Consistency of diarrhea | 11. Over the past 24 hours, what was the consistency of your **worst diarrhea?** |

| | |
|---|---|
| ^{a}Items were scored on a 0-10 scale. | |

Of the Fabry disease-related GI symptoms identified by the experts, abdominal pain, diarrhea, and early satiety were considered to be some of the most important to target during treatment. Of the Fabry disease-related GI symptoms mentioned by patients during the concept elicitation interviews, the most commonly reported symptoms included diarrhea, bloating, and bloating. On a 0-10 scale, where higher scores indicated more bother, worry, and impact, patients most frequently ranked diarrhea, bloating, and constipation as the top symptoms (Table 8).

**Table 8. GI Symptoms and Their Bother, Worry, and Impact**

| **Concept^{a}** | **Patient-rated -reported symptoms^{b}** | **Bother rating^{c}** | **Worry rating^{c}** | **Impact rating^{c}** |
|---|---|---|---|---|
| | | **Mean (SD)** | **Mean (SD)** | **Mean (SD)** |
| Diarrhea | 11/13 | 6.4 (2.0) | 3.5 (2.9) | 5.1 (3.3) |
| Bloating | 9/10 | 4.9 (2.3) | 3.6 (3.2) | 3.3 (3.3) |
| Constipation | 9/10 | 6.4 (3.0) | 5.5 (3.3) | 5.8 (2.8) |
| Cramping | 8/9 | 6.5 (2.3) | 4.4 (2.7) | 6.0 (3.4) |
| Stomach pain | 7/7 | 6.1 (2.9) | 4.9 (1.7) | 5.0 (2.9) |
| Nausea | 5/8 | 6.2 (2.8) | 2.4 (1.9) | 4.0 (3.5) |
| Gas | 4/4 | 3.8 (3.3) | 4.5 (5.3) | 5.0 (4.2) |
| Heartburn | 4/4 | 5.5 (3.7) | 4.5 (3.8) | 3.8 (1.7) |
| Upset stomach | 3/4 | 5.7 (2.1) | 3.0 (2.6) | 5.0 (4.4) |
| Gas pain | 2/3 | 6.5 (0.7) | 3.0 (4.2) | 4.5 (4.9) |
| Frequent bowel movements | 1/2 | 7.0 | 8.0 | 6.0 |
| Burping | 0/1 | - | - | - |
| Vomiting | 1/1 | 8.0 | 4.0 | 5.0 |

| | | | | |
|---|---|---|---|---|
| ^{a}Based on the CEIs. ^{b}The second number indicates the total number of patients that reported experiencing the symptom during the open-ended discussion; the number of patients who had the opportunity to provide a rating for the symptom may be less than the total frequency count for some symptoms as patients were only able to rate symptoms that the interviewer specifically asked about during the interview, prior to the complete analysis of qualitative data. ^{c}Rated on a 0-10 scale where a higher score indicates more bother, worry, or impact. | | | | |

Based on the above, the FABPRO-GI is a new content-validated instrument developed based on literature review, expert advice, and patient perspectives that can be used to assess GI symptoms over a 24-hour recall period in patients with Fabry disease. General understandability, relevance, and comprehensiveness of the FABPRO-GI instrument was validated in a subset of patients with Fabry disease.

### EXAMPLE 3: Baseline Patient Characteristics of followME, a New Patient-centric, Prospective, Observational Fabry Registry That Evaluates Migalastat, ERT, and a Natural History Cohort

This example describes followME, a unique patient-focused, prospective, observational, multinational registry designed to assess the real-world impact of migalastat treatment via safety, effectiveness, and patient-reported outcomes (PROs). The demographics and baseline characteristics of patients currently enrolled in the followME registry was analyzed and is presented below.

### Study Design

Patients receiving migalastat, ERT, or no Fabry-disease specific therapy (natural history control) are enrolled and followed for up to 5 years. Eligibility criteria are presented below:

Inclusion Criteria
Age ≥16 years
Fabry disease diagnosis
eGFR_{CKD-EPI} >30 mL/min/1.73 m²
Provided written, informed consent
Migalastat-Treated Patients
n≈250
ERT-Treated Patients
n≈100
Untreated Patients
n≈100
Prior/Ongoing
Treatment
Criteria
Starting or taking
commercial migalastat
treatment (for <24 months)
at enrollment^{a}
Starting or taking ERT
(for <24 months) at
enrollment
Never received treatment
for Fabry disease
Meet local treatment
guidelines for Fabry
disease
GLA Variant
Criteria
Amenable
Amenable or
non-amenable
Amenable
^{a}Patients receiving migalastat during a clinical trial must have switched to commercial drug at
or before enrollment.

### Methods

Following confirmation of patient consent, physicians collect baseline data for each patient at enrollment in the registry, which include demographics, genotype, and medical history (e.g., Fabry disease history and clinical event history).

The Safety Population includes all patients with Fabry disease who consented to participate in the registry and received at least 1 dose of migalastat or ERT or have never received treatment

Demographics, baseline characteristics, and medical history (e.g., Fabry disease history and clinical event history) are summarized by initial treatment group/

### Results

### Patient Demographics

A total of 144 patients (migalastat: 67; ERT: 35; untreated: 42) from 15 centers in North America and Europe have been enrolled in the registry. Patient demographics are presented in Table 9. Female patients comprise 41.8%, 48.6%, and 88.1% of the migalastat, ERT, and untreated cohorts, respectively.

**Table 9. Demographics of Patients Currently Enrolled in the followME Registry (Safety Population)**

| | | | | | |
|---|---|---|---|---|---|
| **Parameter** | | **Overall N=144** | **Migalastat (n=67)** | **ERT (n=35)** | **Untreated^{a} (n=42)** |

| **Age at enrollment, years** | | | | | |
|---|---|---|---|---|---|
| | Mean (SD) | 50.2 (15.3) | 52.8 (15.5) | 50.4 (13.6) | 45.8 (15.5) |
| | Median (range) | 52.0 (16-81) | 56.0 (16-78) | 52.0 (20-76) | 45.0 (18-81) |

| **Age categories, n (%)** | | | | | |
|---|---|---|---|---|---|
| | ≥ 10-<20 years | 2 (1.4) | 1 (1.5) | 0 (0.0) | 1 (2.4) |
| | ≥20-<30 years | 15 (10.4) | 7 (10.4) | 3 (8.6) | 5 (11.9) |
| | ≥30-<40 years | 22 (15.3) | 6 (9.0) | 4 (11.4) | 12 (28.6) |
| | ≥40-<50 years | 24 (16.7) | 7 (10.4) | 9 (25.7) | 8 (19.0) |
| | ≥50 years | 80 (55.6) | 46 (68.7) | 19 (54.3) | 15 (35.7) |
| | Missing | 1 (0.7) | 0 (0.0) | 0 (0.0) | 1 (2.4) |

| **Gender, n (%)^{b}** | | | | | |
|---|---|---|---|---|---|
| | Male | 61 (42.4) | 38 (56.7) | 18 (51.4) | 5 (11.9) |
| | Female | 82 (56.9) | 28 (41.8) | 17 (48.6) | 37 (88.1) |

| **Ethnicity, n (%)** | | | | | |
|---|---|---|---|---|---|
| | Asian | 3 (2.1) | 1 (1.5) | 1 (2.9) | 1 (2.4) |
| | Black | 0 (0.0) | 0 (0.0) | 0 (0.0) | 0 (0.0) |
| | Hispanic | 26 (18.1) | 14 (20.9) | 5 (14.3) | 7 (16.7) |
| | White | 35 (83.3) | 66 (98.5) | 31 (88.6) | 35 (83.3) |
| | Other | 6 (14.3) | 0 (0.0) | 3 (8.6) | 6 (14.3) |

| | | | | | |
|---|---|---|---|---|---|
| ^{a}Age information is missing for 1 patient in the untreated cohort. ^{b}Gender information is missing for 1 patient in the migalastat cohort. | | | | | |

### Fabry Disease History

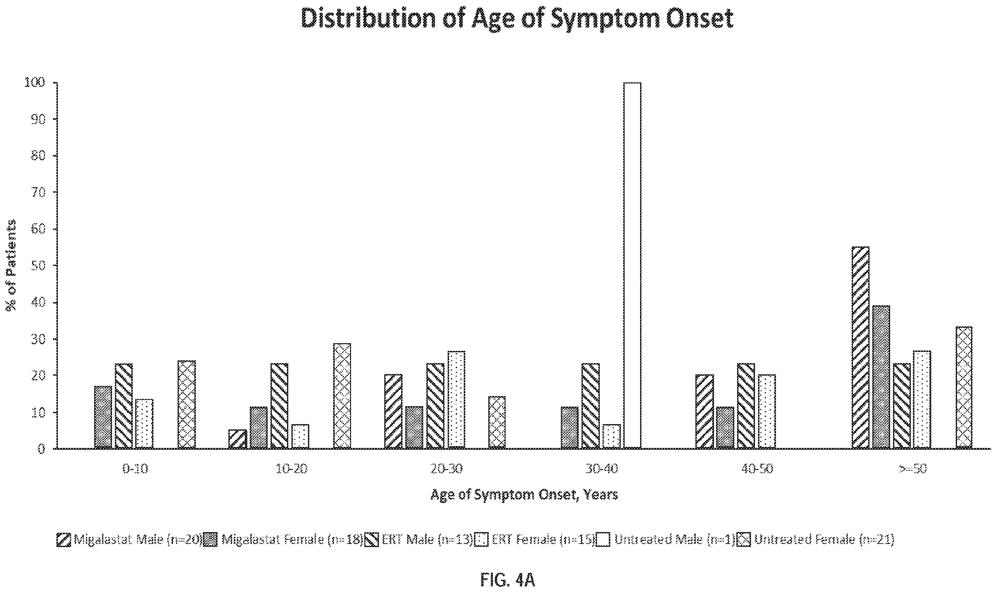

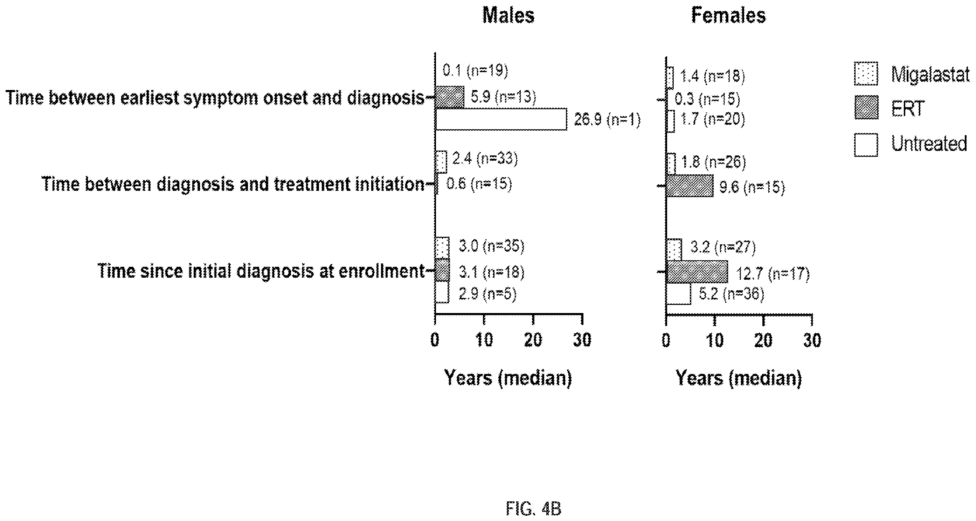

Overall, the median age at symptom onset was 45.5 years in males, and 28 years in females across treatment groups; distribution of age of symptom onset by treatment group and gender is shown in Figure 4A. The median time between earliest symptom onset and diagnosis was 1.2 years in males and 1.4 years in females (Figure 4B).

In the migalastat cohort, the median time between diagnosis and treatment initiation was 2.4 years in males and 1.8 years in females; in the ERT cohort, the median time between diagnosis and treatment initiation was 0.6 years in males and 9.6 years in females (Figure 4B).

### Treatment History

In the migalastat cohort, 3.0% of patients were previously treated with ERT.

In the ERT cohort, 85.7% were treated with ERT prior to enrollment (median [range] ERT treatment duration: 1.3 (0-15.6) years)

### Patient Genotypes

GLA genotype was assessed in 86.6.1%, 82.9%, and 100% of the migalastat, ERT, and untreated cohorts, respectively. 17 variants occurred in more than 1 patient and account for 74.2% of patients with known genotype (Table 10). The most common variant was p.N215S (n=24 [18.8%]), a variant associated with the late-onset phenotype of Fabry disease.

**Table 10. Patient Genotypes Represented by ≥1 Patient in the followME Registry (Safety Population)^{a}**

| | **Amenability** | **Total N=144** | **Migalastat N=67** | **ERT N=35** | **Untreated N=42** |
|---|---|---|---|---|---|
| **p.N215S** | Amenable | 24 | 15 | 2 | 7 |
| **p.R301Q** | Amenable | 10 | 5 | 0 | 5 |
| **p.R301G** | Amenable | 9 | 6 | 2 | 1 |
| **p.R356W** | Amenable | 7 | 2 | 2 | 3 |
| **p.S238N** | Amenable | 7 | 4 | 0 | 3 |
| **p.R118C** | Amenable | 6 | 3 | 1 | 2 |
| **p.T1941** | Amenable | 5 | 2 | 0 | 3 |
| **p.S345P** | Amenable | 5 | 1 | 3 | 1 |
| **p.A143T** | Amenable | 4 | 3 | 0 | 1 |
| **p.R227Q** | Non-Amenable | 3 | 0 | 2 | 1 |
| **p.F113L** | Amenable | 3 | 1 | 2 | 0 |
| **c.1201dupT** | Non-Amenable | 2 | 0 | 0 | 2 |
| **c.1288delT** | Non-Amenable | 2 | 0 | 1 | 1 |
| **p.I232T** | Amenable | 2 | 1 | 1 | 0 |
| **p.I317T** | Amenable | 2 | 1 | 0 | 1 |
| **p.Q386X** | Non-Amenable | 2 | 0 | 2 | 0 |
| **p.R342Q** | Non-Amenable | 2 | 0 | 1 | 1 |

| | | | | | |
|---|---|---|---|---|---|
| ^{a}This is an ongoing study. Genotype has not been confirmed in 10 and 6 patients in the migalastat and ERT groups, respectively. | | | | | |

### Fabry Signs and Symptoms

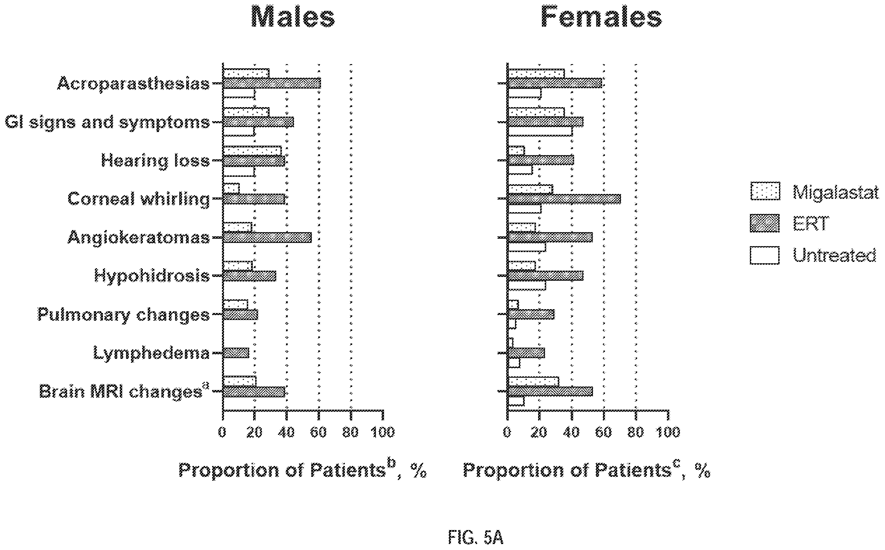

Fabry signs and symptoms reported by patients involve multiple organ systems (Figure 5A). The most commonly reported signs and symptoms among male patients were acroparathesias (37.7%), hearing loss (36.1%), and GI signs and symptoms (32.8%) across treatment groups. Among female patients, the most commonly reported signs and symptoms were GI signs and symptoms (40.2%), corneal whirling (34.1%) and acroparathesias (34.1%).

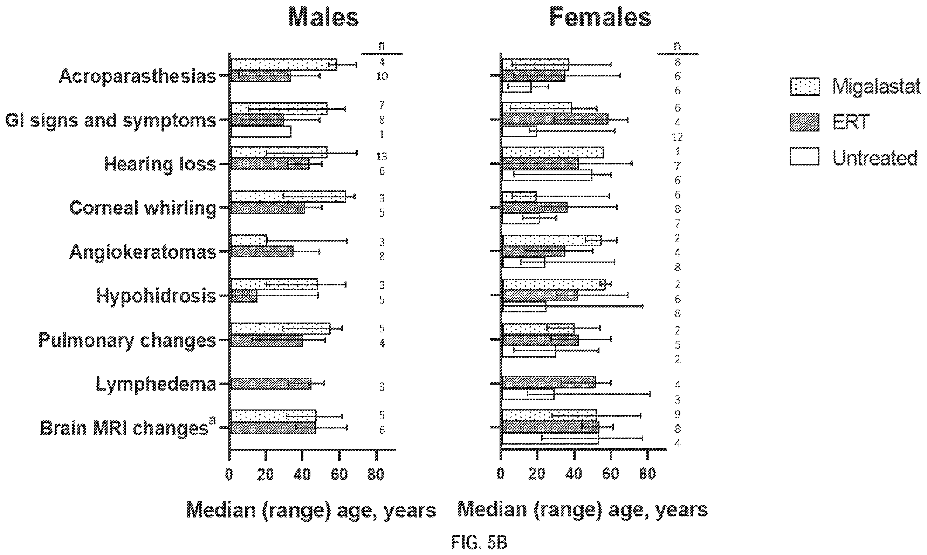

Only a subset of patients provided the age of onset for specific signs and symptoms (Figure 5B). Among male patients with data, hypohidrosis, gastrointestinal signs and symptoms, and acroparasthesias were the earliest signs and symptoms. Among female patients with data, corneal whirling, GI signs and symptoms, and acroparasthesias were the earliest signs and symptoms.

In FIGS 5A and 5B, a = White matter lesions; b = Symptom data were not collected for a small subset of patients: corneal whirling (1 ERT and 1 migalastat patients), angiokeratoma (4 migalastat patients), GI signs and symptoms (1 migalastat patient), MRI changes (4 migalastat patients), lymphedema (7 migalastat patients), pulmonary changes (5 migalastat patients); c = Symptom data of 1 untreated and 1 migalastat patients were not collected for corneal whirling, angiokeratoma, acroparasthesias, hypohidrosis, GI signs and symptoms, hearing loss, lymphedema, and pulmonary changes. Brain MRI history was not collected for 1 untreated patient and 3 migalastat patients.

### Cardiac Manifestations

Summary statistics for left ventricular mass index (LVMi) are shown in Table 11. Untreated female patients had the least cardiac involvement both in terms of mean LVMi and the percentage of patients with left ventricular hypertrophy (LVH). However, ~40% of female patients in the migalastat and ERT groups manifested LVH.

**Table 11. Cardiac Parameters in Patients Enrolled in the followME Registry (Safety Population)**

| | **Male** | | | **Female** | | |
|---|---|---|---|---|---|---|
| **Parameter** | **Migalastat N=38** | **ERT N=18** | **Untreated N=5** | **Migalastat N=28** | **ERT N=17** | **Untreated N=37** |
| **LVM^{a,} g/m²** | | | | | | |
| n | 15 | 12 | 2 | 11 | 10 | 16 |
| Mean (SD) | 96.3 (61.6) | 83.8 (37.5) | 102.4 (63.4) | 90.5 (44.5) | 87.1 (20.7) | 67.7 (21.4) |
| **LVH, n (%)^{b}** | 4 (26.7) | 2 (16.7) | 1 (50) | 4 (36.4) | 4 (40) | 2 (12.5) |

| | | | | | | |
|---|---|---|---|---|---|---|
| ^{a}Calculated based on echocardiography measurements. ^{b}LVH defined as baseline LVMi value >115 g/m² for males and >95 g/m² for females. Percentages were calculated based on the number of patients with echocardiogram. | | | | | | |

### Renal Manifestations

Most patients have renal involvement based on the percentages of patients with detectable urine protein (all except 1 ERT-treated patient) and decreased estimated glomerular filtration rate (33.3% to 55%) in the untreated, ERT, and migalastat cohorts, respectively (Table 12). No patient was undergoing dialysis at enrollment; 1 female ERT patient had a kidney transplant.

**Table 12. Renal Disease in Patients Enrolled in followME Registry (Safety Population)**

| | | **Male** | | | **Female** | | |
|---|---|---|---|---|---|---|---|
| | | **Migalastat N=38** | **ERT N=18** | **Untreated N=5** | **Migalastat N=28** | **ERT N=17** | **Untreated N=37** |
| **eGFR_{CKD-EPI}, mL/min/1.73 m²** | | | | | | | |
| n | | 33 | 17 | 5 | 20 | 13 | 24 |
| Mean (SD) | | 81.7 (29.0) | 91.3 (23.9) | 66.1 (36.8) | 88.5 (15.1) | 91.0 (17.5) | 100.0 (15.3) |
| **eGFR_{CKD-EPI} category, n (%)** | | | | | | | |
| ≥90 mL/min/1.73 m² | | 16 (48.5) | 9 (52.9) | 2 (40.0) | 9 (45.0) | 7 (53.8) | 16 (66.7) |
| ≥60 and <90 mL/min/1.73 m² | | 10 (30.3) | 6 (35.3) | 1 (20.0) | 10 (50.0) | 6 (46.2) | 8 (33.3) |
| ≥30 and <60 mL/min/1.73 m² | | 7 (21.2) | 2 (11.8) | 1 (20.0) | 1 (5.0) | 0 | 0 |

| **Creatinine, mg/dL** | | | | | | | |
|---|---|---|---|---|---|---|---|
| | n | 33 | 16 | 5 | 20 | 15 | 30 |
| | Mean (SD) | 1.1 (0.4) | 1.0 (0.3) | 2.6 (3.6) | 0.8 (0.2) | 1.2 (1.8) | 0.7 (0.1) |

| **Urine protein , mg/dL** | | | | | | | |
|---|---|---|---|---|---|---|---|
| | n | 27 | 14 | 5 | 12 | 10 | 18 |
| | Median (range) | 10.7 (3-1800) | 13.6 (0-100) | 7.1 (4-108) | 6.9 (3-148) | 11.5 (2-43) | 7.4 (3-500) |
| **Detectable Urine Protein, n (%)** | | 27 (100.0) | 13 (92.9) | 5 (100.0) | 12 (100.0) | 10 (100.0) | 18 (100.0) |

### History of Cardiac, Renal, and Cerebrovascular Events

In male patients, prior cardiac events (18.0%) were more common than renal (8.2%) or cerebrovascular events (1.6%); in female patients, prior renal events (7.3%) were more common than cardiac (4.9%) or cerebrovascular events (4.9%) (Table 13).

**Table 13. History of Clinical Events Prior to Enrollment in the followME Registry (Safety Population)**

| | **Male** | | | **Female** | | |
|---|---|---|---|---|---|---|
| | **Migalastat N=38** | **ERT N=18** | **Untreated N=5** | **Migalastat N=28** | **ERT N=17** | **Untreated N=37** |
| **Renal events^{a}, n (%)** | 1 (20) | 1 (5.6) | 3 (7.9) | 5 (17.9) | 0 | 1 (2.7) |
| **Cardiac events^{b}, n (%)** | 7 (18.4) | 4 (22.2) | 0 | 2 (7.1) | 0 | 2 (5.4) |
| **Cerebrovascular events^{c}, n (%)** | 0 | 1 (5.6) | 0 | 4 (14.3) | 0 | 0 |

| | | | | | | |
|---|---|---|---|---|---|---|
| ^{a}Renal events include development of ESRD requiring long-term dialysis or transplant. ^{b}Cardiac events included myocardial infarction; palpitations as a symptom of arrhythmia; symptomatic arrhythmia requiring medication, direct current cardioversion, or interventional procedure (e.g., ablation, pacemaker or defibrillator implantation; unstable angina accompanied by electrocardiographic changes resulting in hospitalization or accompanied by elevated B-type natriuretic peptide; any major cardiac medical procedure (e.g. valve replacement, stent-implantation or transplant. ^{c}Cerebrovascular events included transient ischemic attack and stroke. | | | | | | |

Analysis of baseline characteristics of patients currently enrolled shows that patients in the followME registry are representative of patients with Fabry disease in real-world settings.

Acroparasthesias and GI signs and symptoms are among the most common and earliest Fabry signs and symptoms. Most patients had renal involvement and ~26% of patients experienced LVH.

Compared with male patients, female patients are more likely to be untreated even though some experienced classic Fabry signs and symptoms. Female patients experienced a much longer delay between diagnosis and ERT treatment initiation than male patients

Follow-up of these patients will generate long-term outcome data with migalastat, ERT, and the natural history of Fabry disease that will provide valuable insights regarding real-world experience with migalastat and other treatments for Fabry disease.

The appended claims should not be limited by the specific embodiments set forth in the examples, but should be given the broadest interpretation consistent with the description as a whole.

Patents, patent applications, publications, product descriptions, GenBank Accession Numbers, and protocols are cited throughout this application,

## Claims

1. Migalastat or salt thereof for use in a method of reducing the risk of composite clinical outcomes (CCO) in a female patient having Fabry disease, wherein the CCO comprises renal events, cardiac events, cerebrovascular events and death, the method comprising administering to the patient a formulation comprising an effective amount of migalastat or salt thereof every other day for at least 18 months, wherein the effective amount is about 100 mg to about 150 mg free base equivalent (FBE).

2. Migalastat or salt thereof for use according to claim 1, wherein the renal events comprise one or more of: a decrease in eGFR_{CKD-EPl}≥15 mL/min/1.73 m², with the decreased eGFR <90 mL/min/1.73 m² relative to baseline; or an increase in 24-hour urine protein ≥33%, with elevated protein ≥300 mg relative to baseline.

3. Migalastat or salt thereof for use according to claim 1 or 2, wherein the cardiac events comprise one or more of: myocardial infarction; unstable cardiac angina; new symptomatic arrhythmia requiring antiarrhythmic medication, direct current cardioversion, pacemaker, or defibrillator implantation; or congestive heart failure [New York Association Class III or IV].

4. Migalastat or salt thereof for use according to any one of claims 1-3, wherein the cerebrovascular events comprise one or more of stroke or transient ischemic attack.

5. Migalastat or salt thereof for use according to any one of claims 1-4, wherein the migalastat or salt thereof enhances α-galactosidase A activity.

6. Migalastat or salt thereof for use according to any one of claims 1-5, wherein the patient is administered about 123 mg FBE of the migalastat or salt thereof every other day.

7. Migalastat or salt thereof for use according to any one of claims 1-6, wherein the patient is administered about 123 mg of migalastat free base every other day.

8. Migalastat or salt thereof for use according to any one of claims 1-6, wherein the patient is administered about 150 mg of migalastat hydrochloride every other day.

9. Migalastat or salt thereof for use according to any one of claims 1-8, wherein the formulation comprises an oral dosage form.

10. Migalastat or salt thereof for use according to claim 9, wherein the oral dosage form comprises a tablet, a capsule or a solution.

11. Migalastat or salt thereof for use according to any one of claims 1-10, wherein the migalastat or salt thereof is administered for at least 3 years or for at least 4 years.

12. Migalastat or salt thereof for use according to any one of claims 1-11, wherein the CCO incidence rate for a group of patients on migalastat therapy for 18 months is less than 1.0 per patient•year or less than 0.5 per patient•year.

13. Migalastat or salt thereof for use according to any one of claims 1-12, wherein the patient is an ERT-experienced patient.

14. Migalastat or salt thereof for use according to any one of claims 1-13, wherein the patient has a HEK assay amenable mutation in α-galactosidase A.

## Patentansprüche

1. Migalastat oder Salz davon zur Verwendung in einem Verfahren zur Verringerung des Risikos von zusammengesetzten klinischen Endpunkten (composite clinical outcomes, CCO) bei einer weiblichen Patientin mit Morbus Fabry, wobei die CCO renale Ereignisse, kardiale Ereignisse, zerebrovaskuläre Ereignisse und Tod umfasst, wobei das Verfahren die Verabreichung einer Formulierung, umfassend eine wirksame Menge an Migalastat oder einem Salz davon, an die Patientin jeden zweiten Tag für mindestens 18 Monate umfasst, wobei die wirksame Menge etwa 100 mg bis etwa 150 mg freies Basen Äquivalent (free base equivalent, FBE) beträgt.

2. Migalastat oder Salz zur Verwendung gemäß Anspruch 1, wobei die renalen Ereignisse eines oder mehrere der folgenden umfassen: eine Verringerung der eGFR_{CKD-EPI}≥15 mL/min/1,73 m², mit dem verringerten eGFR <90 mL/min/1,73 m² im Verhältnis zum Ausgangswert; oder eine Erhöhung des 24-Stunden-Urinproteins ≥33 %, mit dem erhöhten Protein ≥300 mg im Verhältnis zum Ausgangswert.

3. Migalastat oder Salz davon zur Verwendung gemäß Anspruch 1 oder 2, wobei die kardialen Ereignisse eines oder mehrere der folgenden umfassen: Herzinfarkt; instabile Herzangina; neue symptomatische Herzrhythmusstörung, die antiarrhythmische Medikation, Gleichstrom-Kardioversion (direct current cardioversion), einen Herzschrittmacher oder eine Defibrillator-Implantation erfordert; oder kongestive Herzinsuffizienz [New York Association Klasse III oder IV].

4. Migalastat oder Salz davon zur Verwendung gemäß irgendeinem der Ansprüche 1-3, wobei die zerebrovaskulären Ereignisse einen oder mehrere von Schlaganfall oder transitorischer ischämischer Attacke umfassen.

5. Migalastat oder Salz davon zur Verwendung gemäß irgendeinem der Ansprüche 1-4, wobei das Migalastat oder Salz davon die α-Galactosidase-A-Aktivität verstärkt.

6. Migalastat oder Salz davon zur Verwendung gemäß irgendeinem der Ansprüche 1-5, wobei dem Patienten jeden zweiten Tag etwa 123 mg FBE des Migalastats oder eines Salzes davon verabreicht werden.

7. Migalastat oder Salz davon zur Verwendung gemäß irgendeinem der Ansprüche 1-6, wobei dem Patienten jeden zweiten Tag etwa 123 mg Migalastat freie Base verabreicht werden.

8. Migalastat oder Salz davon zur Verwendung gemäß irgendeinem der Ansprüche 1-6, wobei dem Patienten jeden zweiten Tag etwa 150 mg Migalastat-Hydrochlorid verabreicht werden.

9. Migalastat oder Salz davon zur Verwendung gemäß irgendeinem der Ansprüche 1-8, wobei die Formulierung eine orale Darreichungsform umfasst.

10. Migalastat oder Salz davon zur Verwendung gemäß Anspruch 9, wobei die orale Darreichungsform eine Tablette, eine Kapsel oder eine Lösung umfasst.

11. Migalastat oder Salz davon zur Verwendung gemäß einem der Ansprüche 1-10, wobei das Migalastat oder ein Salz davon für mindestens 3 Jahre oder für mindestens 4 Jahre verabreicht wird.

12. Migalastat oder Salz davon zur Verwendung gemäß irgendeinem der Ansprüche 1-11, wobei die CCO-Inzidenzrate für eine Gruppe von Patienten, die 18 Monate lang mit Migalastat behandelt werden, weniger als 1,0 pro Patient•Jahr oder weniger als 0,5 pro Patient•Jahr beträgt.

13. Migalastat oder ein Salz davon zur Verwendung gemäß irgendeinem der Ansprüche 1-12, wobei der Patient ein ERT-erfahrener Patient ist.

14. Migalastat oder Salz davon zur Verwendung gemäß irgendeinem der Ansprüche 1-13, wobei der Patient eine HEK-Assay-zugängliche Mutation in α-Galactosidase A aufweist.

## Revendications

1. Migalastat ou sel de celui-ci destiné à être utilisé dans un procédé de réduction du risque de résultats cliniques composites (CCO) chez une patiente présentant la maladie de Fabry, dans lequel les CCO comprennent des événements rénaux, des événements cardiaques, des événements cérébrovasculaires et la mort, le procédé comprenant le fait d'administrer à la patiente une formulation comprenant une quantité efficace de migalastat ou de sel de celui-ci tous les deux jours pendant au moins 18 mois, dans lequel la quantité efficace est d'environ 100 mg à environ 150 mg d'équivalent de base libre (FBE).

2. Migalastat ou sel de celui-ci destiné à être utilisé selon la revendication 1, dans lequel les événements rénaux comprennent un ou plusieurs parmi : une diminution de **l'eGFR_{CKD-EPI}** ≥ 15 mL/min/1,73 m², avec la diminution de l'eGFR < 90 mL/min/1,73 m² par rapport à la référence ; ou une augmentation de la protéinurie sur 24 heures ≥ 33 %, avec un taux élevé de protéines ≥ 300 mg par rapport à la référence.

3. Migalastat ou sel de celui-ci destiné à être utilisé selon la revendication 1 ou la revendication 2, dans lequel les événements cardiaques comprennent un ou plusieurs parmi : infarctus du myocarde ; angine de poitrine instable ; nouvelle arythmie symptomatique nécessitant un traitement antiarythmique, cardioversion électrique externe, implantation d'un stimulateur cardiaque ou d'un défibrillateur ; ou insuffisance cardiaque congestive [Classe III ou IV de la New York Heart Association].

4. Migalastat ou sel de celui-ci destiné à être utilisé selon l'une quelconque des revendications 1 à 3, dans lequel les événements cérébrovasculaires comprennent un ou plusieurs parmi un accident vasculaire cérébral et un accident ischémique transitoire.

5. Migalastat ou sel de celui-ci destiné à être utilisé selon l'une quelconque des revendications 1 à 4, dans lequel le migalastat ou le sel de celui-ci améliore l'activité de l'α-galactosidase A.

6. Migalastat ou sel de celui-ci destiné à être utilisé selon l'une quelconque des revendications 1 à 5, dans lequel il est administré à la patiente environ 123 mg FBE de migalastat ou de sel de celui-ci tous les deux jours.

7. Migalastat ou sel de celui-ci destiné à être utilisé selon l'une quelconque des revendications 1 à 6, dans lequel il est administré à la patiente environ 123 mg de base libre de migalastat tous les deux jours.

8. Migalastat ou sel de celui-ci destiné à être utilisé selon l'une quelconque des revendications 1 à 6, dans lequel la patiente reçoit environ 150 mg de chlorhydrate de migalastat tous les deux jours.

9. Migalastat ou sel de celui-ci destiné à être utilisé selon l'une quelconque des revendications 1 à 8, dans lequel la formulation comprend une forme galénique orale.

10. Migalastat ou sel de celui-ci destiné à être utilisé selon la revendication 9, dans lequel la forme galénique orale comprend un comprimé, une capsule ou une solution.

11. Migalastat ou sel de celui-ci destiné à être utilisé selon l'une quelconque des revendications 1 à 10, dans lequel le migalastat ou le sel de celui-ci est administré pendant au moins 3 ans ou pendant au moins 4 ans.

12. Migalastat ou sel de celui-ci destiné à être utilisé selon l'une quelconque des revendications 1 à 11, dans lequel le taux d'incidence CCO pour un groupe de patientes traitées au migalastat pendant 18 mois est inférieur à 1,0 par patiente/an ou inférieur à 0,5 par patiente/an.

13. Migalastat ou sel de celui-ci destiné à être utilisé selon l'une quelconque des revendications 1 à 12, dans lequel la patiente a déjà reçu une ERT.

14. Migalastat ou sel de celui-ci destiné à être utilisé selon l'une quelconque des revendications 1 à 13, dans lequel la patiente présente une mutation réactive à l'étude HEK dans l'α-galactosidase A.
